# EUROPEAN PATENT SPECIFICATION

(11) **EP 1 510 820 B1**
(45) Date of publication and mention of the grant of the patent: **17.03.2010**
(21) Application number: 04104026.2
(22) Date of filing: 20.08.2004
(51) Int. Cl.: G01N 33/53, G01N 33/574, C12Q 1/68

(54) **Method for detecting medically relevant conditions in a solubilized LBC sample**
Verfahren zum Nachweis von medizinisch relevanten Zuständen in gelösten LBC-Proben
Procédé pour la détection d'états médicalement pertinents dans des échantillons LBC solubilisés

(30) Priority: 25.08.2003 EP 03103218
(43) Date of publication of application: 02.03.2005
(73) Proprietor: MTM Laboratories AG, 69120 Heidelberg (DE)
(72) Inventor: Ridder, Ruediger, 69198 Schriesheim (DE); Reichert, Anja, 69226 Nussloch (DE); Herkert, Matthias, 69120 Heidelberg (DE); Duwe, Alexander, 69120 Heidelberg (DE); Hipfel, Rainer, 72336 Balingen (DE); Martin, Peter, 69251 Gaiberg (DE)

(56) References cited:
- EP-A- 1 388 734
- EP-B1- 11 507 148
- WO-A-02/08764
- WO-A-99/29890
- WO-A-03/057914
- US-A- 5 861 257
- BRULE VAN DEN A J C ET AL: "RAPID DETECTON OF HUMAN PAPILLOMAVIRUS IN CERVICAL SCRAPES BY COMBINED GENERAL PRIMER-MEDIATED AND TYPE-SPECIFIC POLYMERASE CHAIN REACTION" JOURNAL OF CLINICAL MICROBIOLOGY, WASHINGTON, DC, US, vol. 28, no. 12, December 1990 (1990-12), pages 2739-2743, XP009015539 ISSN: 0095-1137
- KLAES R ET AL: "Overexpression of p16INK4a as a specific marker for dysplastic and neoplastic epithelial cells of the cervix uteri" INTERNATIONAL JOURNAL OF CANCER, NEW YORK, NY, US, vol. 92, no. 2, 2001, pages 276-284, XP002225497 ISSN: 0020-7136
- TARKOWSKI ET AL: MOLECULAR DIAGNOSIST, vol. 6, no. 2, 2001, pages 125-130,
- 'DNA and RNA Purification' MASTERPURE COMPLETE DNA AND RNA PURIFICATION KIT, [Online] 2009, EPICENTRE BIOTECHNOLOGY, pages 1 - 3 Retrieved from the Internet: <URL:http://www.epibio/com/item.asp?ID=313>
- BIANCHI ET AL J. CLIN. MICROBIOL. vol. 40, no. 5, 2002, pages 1749 - 1754
- 'Molecular diagnostics: PCR' CHLAMYDIAL DIGNOSIS - AMPLICOR CT/NG PCR - COBAS AMPLICOR, [Online] 2009, pages 1 - 4 Retrieved from the Internet: <URL: www.chlamydiae.com/restricted/docs/labtests /diag>

## Description

The present invention relates to a method for detection of medically relevant conditions in solubilized LBC samples. Said method does not rely on morphological parameters and makes use of biochemical non-cell-based analysis of the presence or absence and or the level of marker molecule in the solubilized LBC sample. The detection of marker molecules is carried out by detection of the presence or absence and or the level of certain proteins, or fragments thereof.

### BACKGROUND OF THE INVENTION

The diagnosis of a large number of medically relevant conditions is nowadays performed using molecular markers as tools. The molecular tools are generally used as one aspect in a complex examination, taking into account a series of different parameters characterizing the samples to be examined.

In medically relevant analysis the morphological examination of samples by cytological means is in common use. Such methods based on morphological characterization of cell based samples are applicable for example in analysis of clinical samples such as body fluids, blood, surgical resections, secretions, swabs or lavages.

Currently diagnostic cytology applications increasingly employ liquid based cytology (in the following shortened to LBC) methods. This is due to the fact that LBC may provide enhanced morphology properties of the cytological preparation and that contaminants such as e.g. mucus or the like may be removed by the LBC procedure. Furthermore LBC enables for storage of unprocessed sample material that may be subjected to various kinds of cytological examinations afterwards. In conventional cytology the sample material is processed to a conventional cytology specimen. The left over could not be preserved. Therefore only one single analysis could be applied to conventional specimens.

One important application of LBC pertains to population wide screening for cervical cancer. However similar methods are applicable for other cancer entities and the respective precursor stages such as e.g. cancers of the urinary system, of the respiratory tract and others.

It is current practice to use LBC specimens for preparation of a monolayer or thin-layer cytology specimen. This specimen is subjected to a cell based analysis. Under certain circumstances immuno-cytochemical reactions are applied to the cytology specimens. However in common practice LBC samples are used for cell based analysis of the specimens. This is also visible from the fact that the arguments for using LBC point to the advantages regarding the cellular morphology and the removal of contaminants.

In screening for cervical cancer for example LBC samples prepared out of swabs are used for detection of neoplastic lesions of the cervix uteri. In the screening procedure lesions of different origin have to be distinguished. Causes for lesions may for example be inflammations (due to infectious agents or physical or chemical damage) or preneoplastic and neoplastic changes. In morphological examinations the lesions of different characteristics are sophisticated to distinguish. Thus, for examination of conventional smears as well as of thin-layer specimens prepared from LBC samples cytologists and pathologists have to be especially trained and even experienced examiners have a high inter- and intra-observer variance in the assessment of a diagnosis based on cytological specimens. In general the result of the examination is based upon the subjective interpretation of diagnostic criteria by the examining pathologist/cytologist. As a result the rate of false positive and false negative results in the screening tests remains unsatisfying high.

Therefore in many cases these cytological examination procedures are supported by the use of molecular markers. Such markers are often used in immuno-cytochemical staining reactions, or in the course of in-situ hybridisation reactions. In the prior art combinations of morphological examinations and immuno-cytochemical staining reactions based on marker molecules, characteristic for different medically relevant states of tissues or cells, may lead to enhanced results. The morphologic examination remains laborious and time consuming and thus expensive, even when supported by the molecular methods, that make the results more reliable. E.g. W00208764 discloses protein and peptide based detection of marker molecules such as HPV E4, MCM protein, and others in cell based applications including histological and cytological preparations. To reduce the cost of diagnostic programs employing cytological examinations a preselection of the samples to be examined by cytological methods should be performed. This would lower the cost and reduce the burden for the cytological examination and would thus contribute to the accuracy of the cytology based diagnosis.

In the art methods for detection of cervical cancer on the basis of solubilized cervical samples are described in Brule, van den, et al.; J. Clin. Microbiol:, Vol 28/12, 1990, pages 2739-2743, WO03/057914 and WO99/29890. Brule et al discloses a method for PCR based detection of HPV nucleic acids in cervical scrapes. WO99/29890 describes a method for detection of HPV mRNA in cervical samples and discloses the possibility of use of LBC sampes as a starting point for such examinations. WO03/057914 also disclosed an mRNA based method for detection of HPV nucleic acids in cervical specimens. All documents disclose the use of the methods in the course of diagnosis of cervical cancer. However all three documents use nucleic acid based detection of HPV expression products as molecular marker and do not indicate any use of protein or peptide marker molecules for similar detection methods.

A method for detection of HPV nucleic acids from LBC samples is disclosed in WO99/29890 by Digene Corp.. This method uses LBC samples as basis for the analysis. Detection of the HPV nucleic acids is performed after lysis of the cells contained in the LBC samples. In this method no normalization of the amount of the LBC sample to be employed in the biochemical non-cell based detection of the HPV nucleic acid, is performed with respect to information obtained from the cytological specimen prepared out of the same LBC sample. The method disclosed by Digene is therefore restricted to mere qualitative measurements. Any biochemical non-cell based quantitative or even semiquantitative method needs information on the composition of the samples obtainable either from biochemical markers or from the microscopic or flow cytometric analysis of the sample.
In EP1388734 a non-cell based method for detection of diseases is disclosed. This method employs a normalization step based on a normalization marker molecule to substitute the information that could be obtained from cytological specimens and thus allows to assess diagnosis. Therefore this method is based on the simultaneous detection of a marker molecule for a disease together with a normalization marker from each specimen. No hint is given in this document as to a method for assessment of diseases from solublized body samples on the basis of onlydisease markers without normalization to a second biochemical marker.
In the present invention the use of LBC samples for the assessment of diagnosis enables for an accurate and comparable way to provide cytological information for the biochemical non-cell based testing. The employment of biochemical normalization with respect to markers indicative for the presence or absence of cells or cell types is omissible. The advantage of using LBC samples in this respect is that the cytologically cell based information is direct related to the homogeneous LBC specimen and thus provides valuable accurate information for use in the evaluation of the biochemical non-cell based test results.

A method for detection of molecular markers on the protein or nucleic acid level from solubilized specimens on the other hand is disclosed in various publications. However no link to the use of LBC samples as a source of the sample specimen in made in this respect. Generally LBC methods are applied in the art to enable for improved morphological evaluation of cytology specimens. The field of application of the LBC samples is therefore indicated only for cytology. Based on the disclosure in the prior art preparation of an LBC sample for subsequent solubilization of the sample for biochemical testing is not disclosed. Moreover the disclosure as to the advantages of LBC procedures teach away from application of LBC samples in any method that is not founded on cellular morphological evaluation of the specimens. According to the inventors findings the use of LBC samples as a source for biochemical non-cell based determination of protein levels in solubilized specimens provides the advantage that the results may be directly compared to a cytological specimen. The protein based biochemical analysis in this respect may serve as a e.g. pre-testing or to provide further information or even to confirm a cytologically equivocal result. In further embodiments the information obtained from the biochemical non-cell based testing may be for the design of the cytological procedures to be applied.

A method for assessment of diagnosis of medically relevant conditions on the basis of solubilized LBC samples, as provided by the present invention could for example serve as for pre-selection of samples to be analysed by means of cytology.

The inventors insight that use of LBC samples as a source of sample material for the development of diagnostic methods for the biochemical non-cell based assessment of diagnosis of medically relevant conditions is another aspect of the present invention. In the art LBC samples are used for development of cell based assay formats. Lysis of the samples in a way as disclosed herein however enables inventors to base the development of the biochemical methods on sample material which is suited to provide information on the patients disease status from other diagnostic procedures on the same sample material. The development method disclosed herein is therefore of great value for achieving effective and reliable kits and in-vitro diagnostic devices. The method for development of kits and in-vitro diagnostic devices as disclosed herein achieves comparability of the results generated by biochemical non-cell based analysis with the cytologically assessed results by means of a normalization. This normalization of the sample for application in the biochemical test format is performed with respect to information on the LBC sample obtainable from the cytological specimen prepared from the LBC sample. Such information comprises e.g. cellularity of the LBC sample, information with respect to volume of the LBC sample, information with respect to mass of the LBC sample or with respect to parameters accessible only via the generation of a thin-layer specimen out of the LBC sample. In this respect the inventors provide by the methods as claimed herein a reliable method for development of kits and in-vitro diagnostic devices on the basis of LBC samples.

### BRIEF DESCRIPTION OF THE INVENTION

The present invention is based on the inventors findings illustrated in the Examples provided herein that assessment of diagnosis of conditions, that are normally (in cell based diagnostic systems) enabled and/or supported by cytological examination procedures, may be performed in solutions from solubilized LBC samples by a method comprising the steps of detecting the level of one or more markers associated with the condition to be diagnosed and diagnosing the presence or absence of a condition in the sample.

The method according to the present invention may for example be applied as a primary screening test in cases, where a cytological examination is normally performed. Using the present invention one may discriminate, if the condition to be diagnosed may be present in the sample. If the solution based diagnosis gives a negative result concerning a particular condition maybe further examination is omissible. In case of positive results ascertaining by classically applicable methods may follow. Thus expensive and time consuming microscopic or other examinations could be avoided by means of an inexpensive rapid biochemical non-cell based testing procedure.

One aspect of the present invention is a method for enhanced diagnosis of medically relevant conditions, wherein the assessment of diagnosis is performed using solutions of solubilized LBC samples. The method for diagnosis disclosed according to the present invention does not rely on morphological parameters but enables for a diagnosis by means of biochemical analysis. The method for assessment of diagnosis of medically relevant conditions according to the present invention employs biochemical non-cell-based analysis of the presence or absence and or the level of marker molecules in solubilized body samples, wherein the body sample is an LBC sample, and wherein the detection of marker molecules is carried out by detection of the presence or absence and or the level of proteins or fragments thereof in said solubilized samples. The marker molecules that may be applied for this method and the medically relevant conditions for which the method can be applied are specified in claim 1.

The samples intended for Liquid Based Cytology processes (in the following denominated as LBC samples) are solubilized in an appropriate lysis medium and are used for in-vitro diagnostic methods for detection of medically relevant conditions from solubilized body samples on the basis of biochemical non-cell-based analysis.

It is an advantage of the (stet) method as disclosed herein that the same specimen, on which the diagnosis for an individual is assessed, may be used for assessment of the biochemical based result. Thus comparability of the biochemical result to the diagnosis is ensured.

### BRIEF DESCRIPTION OF THE DRAWINGS

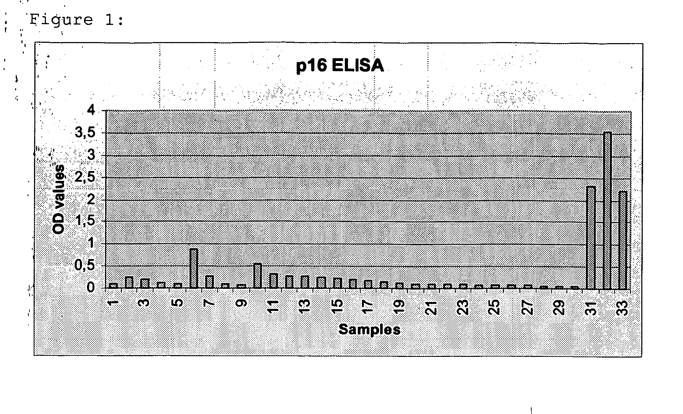

Figure 1 shows the OD values returned in an ELISA test detecting the level of p16^{INK4a} in solubilized cervical samples; for experimental details see Example 1.

### DETAILED DESCRIPTION OF THE INVENTION

Based on the findings of the inventors a method for assessment of diagnosis of medically relevant conditions is performed by biochemical non-cell-based analysis as defined in the appended claims.

According to the invention, cytological examination procedures may be supported or even substituted by the use of molecular markers. Such markers may e.g. be used in immuno-cytochemical staining reactions, or in the course of in-situ hybridization reactions. Combinations of morphological examinations and immuno-cytochemical staining reactions based on marker molecules, characteristic for medically relevant conditions such as e.g. cancers may lead to enhanced results. The morphologic examination remains laborious and time consuming and thus expensive, even when supported by molecular methods that make the results more reliable. Additionally, the diagnosis on a morphologically cell based level is, even when supported by molecular parameters, subject to individual perception of the morphology by individual examiners. Thus the diagnosis is dependent on the person, that performs the examinations.

According to the present invention the use of LBC samples for the assessment of diagnosis may for example provide an accurate and comparable way to provide cytological information for the biochemical non-cell based testing. This may be achieved by employment of normalization of the sample with respect to information obtainable from a cytological specimen prepared out of the same LBC sample. Biochemical normalization with respect to markers indicative for the presence or absence of cells or cell types is omissible in such methods. The advantage of using LBC samples in this respect is that the cytologically cell based information is direct related to the homogeneous LBC specimen and thus provides valuable accurate information for use in the evaluation of the biochemical non-cell based test results.

In the art the field of application of LBC samples is to enable for improved morphological evaluation of cytology specimens. The field of application of the LBC samples is therefore classically indicated only for cytology. According to the present invention the use of LBC samples as a source for biochemical non-cell based determination of protein levels in solubilized specimens provides the opportunity that the results of the biochemical non-cell based testing may be directly compared to a cytological specimen. The protein based biochemical analysis in this respect may serve as a e.g. pre-testing or to provide further information or even to confirm a cytologically equivocal result. In further embodiments the information obtained from the biochemical non-cell based testing may be for the design of the cytological procedures to be applied.

By means of the present invention, it is possible to diagnose medically relevant conditions such as e.g. neoplastic disorders. In particular, precursor stages of cancers can be detected early. It must also be emphasized that it is possible to make a differentiation with respect to benign inflammatory or metaplastic changes of neoplastic disorders. Another characteristic is that the results obtained by a method according to the invention do not rely on subjective evaluation, so that e.g. false-negative results and false-positive results of a Pap test or other cytological preparations can be reduced or avoided. In addition, the present invention distinguishes itself by rapid and simple handling, so that it can be used for extensive screening measures, particularly also in third-world countries. Thus, the present invention represents an important contribution to today's diagnostics of medically relevant conditions.

The inventors could show that molecular markers may be used as diagnostic tools without further support by cell based morphological examinations. Methods for diagnosis of medically relevant conditions on a molecular non-cell based level only, without the support of cell based information, may be applied to solubilized samples such as e.g. LBC samples. The biochemical analysis may serve as stand alone assay or as an adjunct or conjunct testing to a cytological examination of the specimen.

The method of assessment of diagnosis according to the present invention is performed using LBC samples as raw material. The method according to the present invention for analysis of solubilized LBC samples may be applied as an adjunctive or conjunctive test to an established cytological analysis method or as a stand alone test.

LBC samples as used in the context of the present invention are understood to be body samples comprising any cell samples that are preserved in a standard sample collection, storage or transportation medium, known to those of skill in the art such as e.g. commercially available preservation media (formalin solution, Cytyc "PreservCyt" or "CytoLyt", Digene "Universal Collection Medium", Tripath Imaging "Cytorich", etc.). LBC samples accordingly comprise cell samples in any suitable cell preservation medium that may contain a mixture of one or more selected from a group comprising alcohols, aldehydes, ketones, acids, metal-ions or sublimates, ethers etc. for preservation of cellular components. Alcohols include methanol, ethanol, (n- or i-) propanol, (n-, i- or t-) butanol or higher branched or unbranched alcohols. Aldehydes include formaldehyde, acetaldehyde, glutaraldehyde, etc. Ketones such as Acetone may be used. Acids for use in standard sample media include organic acids (acetic acid, trichloro-acetic acid, salicylic acid, picric acid) or inorganic acids such as e.g. chromic acid. Standard sample solutions may comprise metals such as silver, copper, chromium, mercury, osmium, uranium. Solutions of salts such as uranyl-acetate, potassiumbichromate, ammonium sulfate, etc. may be components of preservative media.

LBC samples may be samples of any kind of cells taken for various diagnostic purposes. Currently LBC samples with respect to diagnostics in human healthcare are prepared from any body regions where cytological and/or microbiological testing procedures are indicated or seem to be reasonable. It is believed that for a variety of cytologic specimens LBC samples provide a way that minimizes cell loss and preserves morphologic detail. LBC samples according to the present invention therefore comprise samples obtained as Fine Needle Aspirates. Fine Needle Aspirates may comprise specimens from various sources such as e.g. from breast, thyroid (e.g. from nodules), kidneys, pancreas, prostate, lung, lymph nodes, pleura, neck masses, ovaries, synovia, tumor masses etc.. LBC samples may furthermore be prepared using body fluids. Suitable body fluids comprise a large range of fluids obtainable from the human or animal body comprising but not limited to e.g. ascites, liquor cerebrospinalis, pus or effusions. Effusions wherever in the body they appear may be subjected to LBC. Some examples for effusions are pericardial, pleural, synovial and abdominal effusions. Body fluids to which LBC may be applied comprise furthermore e.g. the fluids present in some tumors or cysts such as e.g. breast cysts, ovary cysts or others. Samples obtainable in liquid form from the body comprise furthermore mucous specimens such as e.g. sputum. LBC is widely applied to any kind of exfoliative cytological specimen. Such exfoliative cytological specimens are obtainable by various methods such as e.g. by any by kind of swab, brushing, scrape, smear etc. Also specimens such as washes, lavages etc. from any body region shall be understood to be exfoliative cytological specimens. Washes and lavages may be obtained from a wide range of body regions including but not limited to mucosal epthelia, the skin, any inner or outer body epithelium or the like. Mucocal epithelia may be e.g. those epithelia of the gastrointestinal tract, of the urinary system, of the anogenital tract, of the respiratory tract, of the rectum, the urethra, the cervix, the vagina, the vulva the oral cavity, the endometrial cavity etc.. The whole range of exfoliative cytological specimens may be subjected to LBC methods.

For the LBC samples used in the method according to the present invention information regarding the cytological procedures may be present or absent. Such information comprises e.g. the volume of an LBC sample needed for preparation of a suitable thin-layer preparation, information on the cellularity or cell content of the LBC specimen, information on the adequacy of the LBC specimen or the underlying sampling procedure, information on the diagnostic information assessed on the basis of a cytological specimen, information on the patient disease and diagnosis etc..

In a method according to the present invention the LBC sample obtained is used either in its entirety or only in parts. In certain embodiments of the invention the total volume of an LBC sample is used. In another embodiment the total number of cells contained in the sample is used. In yet another embodiment only a fraction of the total volume or of the total number of cells contained in the original LBC sample is used.

In the method a normalization of the LBC sample may be applied (this is not part of the claimed invention). In certain embodiments a normalization of the LBC sample may be applied to ensure the presence of a comparable amount of cells in the biochemical non-cell based analysis processes performed using the solubilized LBC sample. This may e.g. be achieved by normalizing the volume of the LBC sample with respect to the volume of said sample necessary for preparation of an appropriate thin-layer specimen. (Preparation of the thin-layer specimen may be performed by any suitable method known to those of skill in the art such as e.g. employing centrifugation, a ThinPrep™ processor or the like.) In this case the volume of the fraction of the LBC sample for employment in the biochemical non-cell based analysis process according to the present invention may be variable for the different samples. In certain further embodiments the normalization of the LBC sample is performed with respect to the volume of the sample subjected to the testing procedure. In this case the amount of cells present in the fraction of the LBC sample for employment in the development process according to the present invention may be variable.

The expression "body sample" as used herein comprises any body samples of any kind and nature. Body samples as used in the context of the present invention comprises the term "LBC sample" as a special embodiment of a body sample. Examples of such body samples are secretions, swabs, lavages, body fluids, semen, cell- and tissue-samples, blood, smears, sputum, urine, stool, liquor cerebrospinalis (short denominated as "liquor" herein) , bile, gastrointestinal secretions, lymph, bone marrow, aspirates and biopsies of organs such as needle or punch biopsies and (fine)-needle aspirates. In particular, smears, swabs and biopsies are indicated when the detection of anogenital cancers, e.g. cervical cancers, is concerned. The term biopsies as used throughout this text shall comprise all kind of biopsies known to those of skill in the art. Thus biopsies as used in the context of the present invention may comprise e.g. resection samples of tumors, tissue samples prepared by endoscopic means or punch- or needle-biopsies of organs. Biopsies comprises specimens obtained by several different methods such as cold knife biopsies, LEEP (loop electrocautery excisional procedure) biopsies, etc..

Furthermore, body samples that have been subjected to cell lysing conditions immediately after obtaining the samples may be used in the methods disclosed herein. Inventors have found a number of robust, fast and easy ways to preserve molecular properties of samples, in which the morphological information of samples is lost. Samples may be e.g. prepared in a reproducible and easy to store and to transport form by solubilizing the cellular components of the raw sample in a suitable lysis medium immediately after or even during obtaining the sample. Body fluids may directly be transferred from the body of an individual to a medium containing suitable detergents and preservative substances. Furthermore, tissue samples may immediately be transferred to denaturing lysis conditions (eventually supported by physical forces) and be thus preserved. Using appropriate ingredients in the lysis medium, the molecular components of the original sample may be preserved, and no degradation may occur. The degradation by enzymatic activities may for example be minimized by the use of enzyme inhibitors. Thus, a solution of test samples in said lysis medium may represent the molecular properties of a test sample at the time of solubilization.

Generally, the lysis medium included in a kit according to the present invention may be any suitable solvent known to those of skill in the art. The lysis medium for use in the kit may, for example, be organic or aqueous solutions of chaotropic agents such as e.g. urea, GuaSCN, Formamid, of detergents such as anionic detergents (e.g. SDS, N-lauryl sarcosine, sodium deoxycholate, alkyl-aryl sulphonates, long chain (fatty) alcohol sulphates, olefine sulphates and sulphonates, alpha olefine sulphates and sulphonates, sulphated monoglycerides, sulphated ethers, sulphosuccinates, alkane sulphonates, phosphate esters, alkyl isethionates, sucrose esters), cationic detergents (e.g. cetyl trimethylammonium chloride), non-ionic detergents (e.g. Tween 20, Nonidet P-40, Triton X-100, NP-40, Igepal CA-630, N-Octyl-Glucosid) or amphoteric detergents (e.g CHAPS, 3-Dodecyl-dimethylammonio-propane-1-sulfonate, Lauryldimethylamine oxide) and/or of alkali hydroxides such as e.g. NaOH or KOH. In certain embodiments, where lysis of cells may be achieved without the use of detergents, hyper- or hypotonic solutions or buffers or simply water or an organic liquid may be used as solvent. Any liquid, that is suited to solubilize the cellular components of body samples in total or in parts may be regarded as a lysis medium as used herein. Thus lysis media as used herein need not contain buffer substances or have buffer capacity.

Generally any suitable liquid may be used as a solvent in the lysis medium of the present invention. The liquid may be organic or inorganic and may be a pure liquid, a mixture of liquids or a solution of substances in the liquid and may contain additional substances to enhance the properties of the solvent. In certain embodiments, where lysis of cells may be achieved without the use of detergents, hyper- or hypotonic solutions or buffers or simply water or an organic liquid may be used as solvent. Any liquid, that is suited to solubilize the cellular components of body samples in total or in parts may be regarded as a lysis medium as used herein. Thus lysis media as used herein need not contain buffer substances or have buffer capacity. However in certain embodiments of the invention the lysis media may have buffer capacity and may contain buffer substances.

In one embodiment, the lysis medium is designed, so that cells, cell debris, nucleic acids, polypeptides, lipids and other biomolecules potentially present in the raw sample are solubilized. In further embodiments of the present invention, the solvent may be designed to assure differential solubilization of specific components of the body sample, leaving other components unsolubilized.

The lysis medium for solubilizing the body sample according to the present invention may furthermore comprise one or more agents that prevent the degradation of components within the raw samples. Such components may for example comprise enzyme inhibitors such as proteinase inhibitors, RNAse inhibitors, DNAse inhibitors, etc. In one embodiment of the present invention, the sample is lysed directly in the form obtained from test-individuals. Proteinase inhibitors may e.g. comprise inhibitors of serine proteinases, inhibitors of cysteine proteinases, inhibitors of aspartic proteinases, inhibitors of metally proteinases, inhibitors of acidic proteinases, inhibitors of alkaline proteinases or inhibitors of neutral proteinases. In certain embodiments of the present invention the inhibition of enzymes may be achieved by chemical means such as e.g. denaturation of the enzymes by means of salt concentration, pH, chaotropic agents or the like.

In certain embodiments of the invention in order to obtain optimal results of the assay, the pH of a lysis medium that can be directly applied to the assay system is around neutral. In further embodiments the pH of the lysis medium is within the range of 4 to 10. In certain other embodiments, the pH is in a range from 5 to 9. In a preferred embodiment, the pH is in a range from 6 to 8. In a more preferred embodiment, the pH is in the range from 6.5 to 7.5.

Examples of lysis media may for example be selected from the compositions given in Table 1.

**Table 1**

| ***Lysis medium*** | **solubilization of p16^{INK4a} in Western blot** | **compatibility with Elisa** |
|---|---|---|
| | | |

| **Detergents:** | | |
|---|---|---|
| 0.1-1% SDS | + | +/- |
| 0.2-3% SDS | + | < 0.5 % |
| 0.2-3% DOC | ++ | +/- |
| 0.1-1% n-Octylglycoside | + | yes |
| 0.1-3% Triton x-100% | + | yes |
| 0.1-1% Chaps | + | nd |

| **Detergent-Mix:** | | |
|---|---|---|
| RIPA (1%NP40, 0.5%DOC, 0.1%SDS, PBS) | | |
| 40-100% | ++ | yes |

| SOX (0.5% DOC, 0.5% n-Octylglycoside) | | |
|---|---|---|
| 40-100% | + | yes |
| mtm lysis medium (3% Tritonx-100, 0.4 % SDS, PBS) | ++ | yes |

| **Commerical lysis media:** | | |
|---|---|---|
| Dynal (Dynal, Oslo, Norway) | ++ | yes |
| M-PER/B-PER (Pierce, Rockford, IL) | ++ | yes |

| **Miscellaneous:** | | |
|---|---|---|
| 0.5-8 M urea in PBS | +++ | Compatible < 2 M |
| Lämmli sample buffer | +++ | no |
| 10-80% DMSO | +++ | no |
| 10-80 % Formamide | nd | no |
| 50-70% formic acid | ++ | no |
| PBS | +/- | yes |
| Citrate buffer pH 6.0 | +/- | yes |
| 500 mM NaCl in Phosphate buffer | +/- | yes |

| | | |
|---|---|---|
| nd: not determined; +/-: poor; +: good; ++: very good; +++: excellent; | | |

In certain situations, the marker molecules can be degraded in the solubilized samples and may thus not be detected. This is particularly true, if the samples are directly transferred to a lysing medium and stored therein for a certain period of time. To prevent degradation, lysis medium may furthermore comprise one or more agents that prevent the degradation of components within the raw samples. Such components may for example comprise enzyme inhibitors such as proteinase inhibitors, RNAse inhibitors, DNAse inhibitors, etc. The inhibitors may e.g. comprise proteinase inhibitors selected from the compositions given in Table 2.

**Table 2:**

| **Inhibitor** | **class of inhibited concentration proteinase** | | **Solubility in water** | **stability in water** | **p16^{INK4a}** |
|---|---|---|---|---|---|
| | | | | | **stabilization in mtm** |
| | | | | | **Lysis medium** |
| Aprotinin | Serine | 0.6-2 µg/ml | Very good | good | no |
| Benzamidine | Serine | 0.5-4 mM | good | good | no |
| Bestatin | Aminopeptidases | 1-10 µM | good | good | no |
| Calpeptin | Cysteine | 0.3-1 µM | good | good | no |
| Cystatin | Cysteine | 1 µM | good | good | no |
| E-64 | Cysteine | 1-10 µM | good | good | no |
| EDTA | Metallo | 0.5-5 mM | good | good | no |
| Elastatinal | Serine | 0.5-2 µg/ml | poor | good | no |
| EST | Cysteine | 20-50 µg/ml | bad | poor | no |
| Fetal calf serum | all classes | 10% | good | good | yes |
| Leupeptin | Serine/Cysteine | 10-100 µM | good | good | no |
| a2- | all classes | 1 µM | good | good | no |
| Macroglobulin | | | | | |
| NCO-700 | Cysteine | 0.5-100 mM | poor | poor | no |
| Pefabloc= | Serine | 0.2-10 µM | good | very poor | yes |
| AEBSF | | | | | |
| Pepstatin A | Aspartic | 1 µM | bad | poor | no |
| PMSF | Serine | 0.2-10 µM | bad | very poor | yes |
| o-Phenanthroline | Metallo | 1-10 mM | bad | poor | no |

DNase and RNase inhibitors are known to those of skill in the art and may be applied under suitable condition for use in a lysis medium according to the present invention.
For stabilization purpose, the lysis medium may also comprise bulk protein (e.g. albumin such as bovine serum albumin or calf serum albumin or other bulk proteins) to compete in degradation with the sample proteins. The bulk proteins may e.g. be present in combination with proteinase inhibitors or may be added instead of proteinase inhibitors. In one embodiment, the solvent may be selected to be compatible with the assay (e.g. ELISA) performance, so that solubilized samples may directly be applied to the assay.

In some embodiments of the present invention, the lysis medium may be tailored in order to enable for the setting of a specific cut-off value.

The method of detection disclosed herein in this respect pertains to detection of marker molecules such as proteins or peptides and the respective fragments thereof. In certain embodiments the detection of marker molecules is carried out by immunochemical detection of the presence or absence and or the level of proteins, peptides or fragments thereof in said solubilized samples. The marker molecules that may be applied for this method are disclosed as marker molecules characteristic for medically relevant conditions.

The detection of said marker molecules in the course of a biochemical non-cell-based analysis may be carried out in solution or using reagents fixed to a solid phase. In certain embodiments of the present invention the detection of the marker molecules is performed from a solution of dissolved body samples. Therefore detection may be carried out in solution or using reagents fixed to a solid phase. A solid phase as used in the context of the present invention may comprise various embodiments of solid substances such as planar surfaces, particles (including micro-, nano-particles or even smaller particles). In certain embodiments particles may be provided as beads, colloids or the like. The fixation of reagents to the solid phase in a test kit or an in-vitro diagnostic device may be effected via direct fixation or via indirect fixation. Direct fixation may e.g. be effected by covalent or non-covalent binding or association to surfaces. Indirect fixation may be effected through binding of the reagents (e.g. antibodies, probes etc.) to agents which themselves are directly fixed to solid phases. Such agents may comprise antibodies or other binding agents like streptavidin, biotin or the like. The detection of one or more molecular markers may be performed in a single reaction mixture or in two or more separate reaction mixtures. The detection reactions for several marker molecules may for example be performed simultaneously in multi-well reaction vessels or as the case may be on one single or two or more separate test strips. The markers characteristic for the cell proliferative disorders may be detected using reagents that specifically recognize these molecules. The detection reaction in case more than one marker are to be detected may comprise one or more further reactions with detecting agents either recognizing the initial marker molecules or preferably recognizing the prior molecules (e.g. primary antibodies) used to recognize the initial markers. The detection reaction further may comprise a reporter reaction indicating the level of the markers characteristic for cell proliferative disorders.

The term diagnosis as used according to the present invention generally comprises any kind of assessment of the presence of absence of a medically relevant condition. Diagnosis thus comprises processes such as screening for the predisposition for a medically relevant condition, screening for the precursor of a medically relevant condition, screening for a medically relevant condition, clinical or pathological diagnosis of a medically relevant condition, etc.. Diagnosis or assessment of diagnosis as used herein may furthermore comprise assessment of prognosis or provision of information for stratification of patient therapy on the basis of the biochemical non-cell based testing. Diagnosis of medically relevant conditions as used herein may comprise examination of any condition, that is detectable on a cytological, histological, biochemical or molecular biological level, that may be useful in respect to the human health and/or body. Such examinations may comprise e.g. medically diagnostic methods and research studies in life sciences. In one embodiment of the invention, the method is used for diagnosis of medically relevant conditions such as e.g. diseases. Such diseases may for example comprise disorders characterized by non-wild type proliferation of cells or tissues.

In one embodiment, the diagnosis pertains to diagnosis of neoplastic disorders and their precursor stages, to monitoring of the disease course in neoplastic disorders, to assessment of prognosis of neoplastic disorders and to detection of disseminated tumor cells e.g. in the course of minimal residual disease diagnosis. The method according to the present invention may for example be used in the course of clinical or pathological diagnosis of cancers and their precursor stages or in routine screening tests as performed for particular neoplastic disorders such as for example for examination of swabs e.g. in screening tests for cervical lesions, of bronchial lavages for lung cancer or of stool for lesions of the gastrointestinal tract, e.g. colorectal lesions.

Medically relevant conditions as used according to the present invention may for example be compositions of tissues, body fluids, secretions, washes or swabs. Such conditions may for example comprise the cellular composition of body fluids, such as the composition of blood, the composition of liquor cerebrospinalis or the composition of semen. In this context the compositions shall be for example the presence or absence of particular cell types (e.g. pathogens, such as, viruses etc., , neoplastic and/or dysplastic cells etc.), the presence or absence of differentiation patterns of particular cell types, the total number of a particular cell types (e.g. erythrocytes, leucocytes, sperm, etc.), the total number of all cells of any cell types or the fraction of cells of particular other characteristics present or absent in the sample.

Medically relevant conditions according to the present invention are neoplastic disorders, i. e. tumors. comprise tumors of the respiratory tract, tumors of the anogenital tract, of the gastrointestinal tract, or tumors of the urinary system,

Tumors may comprise for example neoplasms such as benign and malignant tumors, carcinomas, sarcomas, or dysplasias.
Tumors of the anogenital tract may comprise cancer of the perianal and the scrotal skin, cervical cancer, cancer of the vulva, cancer of the vagina, cancer of the penis, cancer of the anus, etc. Cervical cancer may comprise squamous lesions, glandular lesions or other epithelial tumors. Squamous lesions comprise, e.g., cervical intraepithelial neoplasias (mild, moderate and severe dysplasia), carcinoma in-situ, squamous cell carcinoma (e.g., keratinizing, nonkeratinizing, verrucous, warty, papillary, lymphoepithelioma-like). Glandular lesions may comprise atypical hyperplasias, adenocarcinoma in-situ, andenocarcinoma (such as, e.g., mucinous, endometrioid, clear cell, adenoma malignum, papillary, serous or mesonephric adenocarcinoma). Other epithelial tumors may comprise adenosquamous carcinoma, glassy cell carcinoma, adenoid cystic carcinoma, adenoid basal carcinoma, carcinoid tumor, small cell carcinoma and undifferentiated carcinoma. For more detailed information, confer "Kurman, R., Norris, H., et al., Tumors of the Cervix, Vagina, and Vulva, Atlas of Tumor Pathology, 1992, AFIP," the contents of which shall be incorporated herein by reference.

Gastrointestinal tumors may comprise colon cancer, cancer of the colon ascendens, of the colon descendens, of the colon transversum, of the sigmoidum, of the rectum, cancer of the small intestine, cancer of the jejunum, cancer of the duodenum, gastric cancer, oesophageal cancer, liver cancer, cancer of the bile, cancer of the biliary system, pancreatic cancer, etc. A comprehensive overview over gastrointestinal lesions is given in "Hamilton Sr, Aaltonen LA (Eds.): World Health Organization Classification of Tumours, Pathology and Genetics of Tumors of the Digestive System, IARC Press: Lyon 2000," which shall be incorporated herein by reference.

Tumors of the respiratory tract may comprise any malignant condition of the respiratory tract such as, e.g., cancer of the lung, the alveoles, the bronchioles, the bronchial tree and the broncus, the nasopharyngeal space, the oral cavity, the pharynx, the nasal cavity and the paranasal sinus. Lung cancer such as small cell lung cancer, non-small cell lung cancer, squamous cell lung carcinoma, small cell lung carcinoma, adenocarcinoma of the lung, large cell lung carcinoma, adeno-squamous lung carcinoma, carcinoid tumor of the lung, broncheal gland tumor or (malignant) mesothelioma. An overview over tumors of the respiratory tract may be found in Colby TV, et al.: Tumors of the Lower RespiratoryTract, Atlas of Tumor Pathology, Third Series, Fascicle 13, AFIP: Washington 1995," which shall be incorporated herein by reference.

Tumors of the urinary system may comprise bladder cancer, cancer of the kidney, renal pelvis, cancer of the ureters and cancer of the urethra, etc. Tumors of the reproductive system may comprise cancer and precursory stages thereof of the ovary, the uterus, the testis, the prostate, the epididymis, etc. In one embodiment of the invention, the cancer is e.g. cervical cancer, colon cancer, gastric cancer, breast cancer, bladder cancer, lung cancer, cancer of the oral cavity etc.

Neoplastic disorders to which the methods of the present invention may be applied comprise for example, neoplastic lesions of the respiratory tract, of the urinary system, of the gastrointestinal tract of the anogenital tract, neoplastic disorders associated with HPV infection and others. They may be cancers of the respiratory tract, the urinary system, the reproductive tract or anogenital cancers, HPV associated cancers and particularly the cervical cancer. In connection with the latter, its precursor stages, e.g. cervical intraepithelial neoplasias (CIN1-3), carcinomas in situ (CIS), etc., have to be mentioned particularly. The term "precursor stages" in all it's grammatical forms as used herein comprises all precursor stages and precursors of cancers or any other malignancies. With respect to cervical cancer precursor or preliminary stages as used herein may e.g. refer to stages of cervical intraepithelial neoplasias as identified by appropriate classification systems such as e.g. the CIN classification (CIN 1 - CIN 3) the PAP classification (PAP I - PAP V) or the Bethesda Classification (NILM, LSIL, HSIL).

In certain embodiments of the invention neoplastic disorder shall refer generally to HPV associated neoplastic disorders. The invention in this respect is applicable to neoplastic disorders associated with HPV and especially high risk HPV types and mucosal HPV types. The high risk HPV may comprise HPV subtypes such as e.g. HPV 16, 18, 31, 33, 35, 39, 45, 51, 52, 56 and 58. Markers for HPV infection may e.g. comprise HPV expression products of HPV genes L1, L2, E2, E4, E5, E6 or E7.

The methods according to the present invention also apply to precursor stages of the lesions, tumors or cancers.

In one embodiment, the method pertains to the detection of disseminated tumor cells or metastases.

Marker molecules as used in the application shall all times refer to marker molecules characteristic for medically relevant conditions. The terms "marker molecule" or "marker molecule characteristic for medically relevant conditions" in all their grammatical forms as used in the context of the present invention refers to nucleic acid as well as (poly)peptide molecules. Such marker molecules thus comprises e.g. RNA (mRNA, hnRNA, etc.), DNA (cDNA, genomic DNA, etc.), proteins, polypeptides, proteoglycans, glycoproteins and the respective fragments of these molecules.

A level of a marker molecule as used herein refers to a semiquantitative as well as a quantitative value regarding the amount of the respective marker present in a sample. A quantitative value may e.g. be represented in terms of a concentration. A semiquantitative value may be expressed in terms of a scale of levels e.g. undetectable levels, low levels, intermediate levels, high levels or any other suitable mode. The level of a marker may also be represented in terms of a dependent parameter such as the intensity of a signal generated in an assay format in response to the presence of a marker molecule.

A "probe" (used interchangeably with the term "binding agent" within the present invention) for the detection of the marker molecules as used in the context of the present application shall be any molecule, that specifically binds to said marker molecules. The probe may for example be an antigen binding agent such as antibodies (monoclonal or polyclonal), antibody fragments or artificial molecules comprising antigen binding epitopes, DNA or RNA binding molecules such as proteins or nucleic acids. Nucleic acids binding to other nucleic acids may for example be peptide nucleic acids (PNAs) or oligonucleotides (RNA, DNA, PNA, artificial nucleic acids, etc.) for detection purposes or primers. In certain embodiments even larger nucleotide molecules may be applied for hybridization reactions. A molecule is said to recognize another molecule if it specifically interacts with that molecule. Specific interaction may for example be specific binding to or of the other molecule. The term "antibody" in all its grammatical forms shall in the context of the present invention refer generally to antigen binding molecules including but not limited to monoclonal and polyclonal antibodies, fragments of antibodies, antigen binding epitopes, mini-antibodies, peptidomimetics with antigen-binding properties, anticalines and diabodies.

A molecule is said to recognize another molecule if it specifically interacts with that molecule. Specific interaction may for example be specific binding to or of the other molecule.

According to the invention, the detection of the level of marker molecules is carried out by determining the level of expression of a protein. The determination of the marker molecules on the protein level may for example be carried out in a reaction comprising a binding agent specific for the detection of the marker molecules. These binding agents may comprise for example antibodies and antigen-binding fragments, bifunctional hybrid antibodies, peptidomimetics containing minimal antigen-binding epitopes etc. The binding agents may be used in many different detection techniques for example in western-blot, ELISA, RIA, EIA, flow through assay, lateral flow assay, latex-agglutination, immunochromatographic strips or immuno-precipitation. Generally binding agent based detection may be carried out as well in vitro as directly in situ for example in the course of an immunocytochemical staining reaction. Any other method suitable for determining the amount of particular polypeptides in solutions of biological samples can be used according to the present invention.

Applicable formats for the detection reaction according to the present invention may be blotting techniques, such as Western-Blot, Southem-blot, Northern-blot. The blotting techniques are known to those of ordinary skill in the art and may be performed for example as electro-blots, semidry-blots, vacuum-blots or dot-blots. Furthermore immunological methods for detection of molecules may be applied, such as for example immunoprecipitation or immunological assays, such as EIA, ELISA, RIA, FIA (fluorescent immunoassay) lateral flow assays (using porous members or capillaries), immunochromatographic strips, flow through assays, latex agglutination assays etc. Immunoassays for use in the invention may comprise competitive as well as non-competitive immunoassays, such as sandwich assays.

In certain embodiments of the present invention immunochemical testing may be performed using a testing device for clinical laboratories. Such testing device may comprise any device suitable for immunochemical testing including any format such as e.g. Point of care testing devices as well as bench top or laboratory devices. The devices may be e.g. provided as open or closed platform systems. The system may be based on any suitable methodology such as e.g. employing microtiter plates, multiwell plates, flow through or lateral flow systems, microchip or array based systems or bead or membrane based systems. The detection methods employed may comprise any methods known to those of skill in the art useful for immunochemical detection reactions. Such detection systems may be e.g. luminescence systems (electroluminescence, bioluminescence, photoluminescence, radioluminescence, chemiluminescence, electrochemoluminescence), fluorescence based systems, conductivity based detection systems, radiation (light, UV, X-ray, gamma etc.) or any other known method.

The method for detection of the level of the marker molecules is in one embodiment of the present invention any method, which is suited to detect (small) amounts of specific molecules in biological samples. Furthermore any method for detection of the marker molecules irrespective of the sensitivity may be applied. The detection reaction according to the present invention comprises detection reactions on the level of polypeptides. In one embodiment of the invention, the detection of the marker molecules may comprise the detection of particular splicing variants. In another embodiment of the present invention, the detection method may comprise the detection of modifications of marker molecules such as phosphorylation or glycosylation etc of polypeptides in samples.

The reporter reaction may be any event producing a signal in response to the presence of the marker or to the binding of a specific probe to the marker. For example, a reaction producing a colored compound, a fluorescent compound, a light emitting compound, a radiation emitting compound, or the concentration of one or more of these compounds to a detectable concentration in a predefined area of a testing device may serve as reporter reaction.

The reporter reaction may be for example a reaction producing a colored compound. In one embodiment of the present invention the reporter substances correlated to the particular markers develop different colors. In another embodiment, one reporter may be a molecule quenching the signal produced by the reporter molecule specific for another marker, characteristic for the medically relevant condition, in dependence on the level of the respective marker present in the sample. In yet another embodiment the reporter reactions may produce fluorescent dyes with differing wavelength characteristics. In a further embodiment of the present invention the reporter reaction may comprise light emitting reactions with different wavelength characteristics for the reporter substances specific for either marker to be detected. In another embodiment of the present invention the reporter reaction may comprise the emission of radioactive radiation and additional methods for visualizing or quantifying the radiation. In one embodiment, the different marker molecules may be recognized by agents, that bear radio-nuclides emitting radiation with different energetic properties, so that the signals referring to marker molecules could be distinguished.

The preparation of a sample for use in a method as disclosed herein may also comprise several steps of further preparations of the sample, such as separation of insoluble components, isolation of polypeptides or nucleic acids, preparation of solid phase fixed peptides or nucleic acids or preparation of beads, membranes or slides to which the molecules to be determined are coupled covalently or non-covalently.

The expression "determining the overexpression of marker molecules " comprises any methods which are suited for detecting the expression of marker molecule proteins. In order to determine an overexpression, the body sample to be examined may be compared with a corresponding body sample which originates from a healthy person or from a non-diseased region of the respective organ. Such a sample can be present in a standardized form.

The comparison with normal healthy body samples may be achieved by different methods. In one embodiment of the present invention, the comparison may be performed directly by including a control reaction with non-diseased tissue or cell sample. This non-diseased tissue or cell samples may be provided from a healthy person or from non-diseased regions of the human subject under examination or from cell culture cells known to show the properties of non-diseased cells with respect to marker molecule expression. In another embodiment, the comparison may be performed indirectly by comparing the level of marker molecules within the sample under investigation to a level of said marker molecule known to be present in normal healthy samples. The knowledge about the level for normal healthy tissue or cell samples may be derived from a representative number of testings or from scientific publications providing information the expression level of said marker molecule in normal healthy cells. Comparison may be performed by employing a value for the concentration of the marker molecule; otherwise a characteristic value depending on the protein concentration such as the optical density under defined reaction conditions may be employed. Otherwise the known value may be represented by a surrogate control such as a peptide or a recombinant protein. E.g. the level of p16^{INK4a} present in normal healthy samples may be represented by a control sample of a recombinant protein or a peptide in the testing procedure.

Generally, the comparison of the level present in the sample under investigation may be performed with respect to a value determined in each single testing procedure or to a predetermined value. The predetermined value may be determined for the testing procedure globally. Otherwise, the value may be valid only for a certain lot of testing reagents. For example, the reference value may be valid for a defined calibration period only and may be defined upon calibration of the testing process.

For example the level of marker molecule in a healthy human cervical sample can be determined from a standardized sample solution. A standardized sample solution may comprise a solution of a solubilized pool of normal cell or normal tissue samples. The sample pool may, e.g., be a pool of cytological specimens with pre-assessed normal diagnosis from a screening population, or a pool of normal cells obtained from histological specimens. Furthermore, a pool of normal cells may be obtained from tissue culture of normal cervical epithelial cells. The sample solution may, e.g., be standardized with respect to the content of cells per ml sample solution. Any other parameter for standardization may be applied. The sample solution may e.g. be provided in a standardized form to ensure stability and reproducibility of the test results. In certain embodiments such solution may be provided as a component of the kit for comparison or calibration purposes.

In certain embodiments, the step of comparing the level of molecules present in a patient sample to a level known to be present in a normal healthy body sample is embodied as employing a cut-off value or threshold value for the concentration of the respective marker molecule. The cut-off in this context is a value (for example a concentration of marker molecule protein given in e.g. mg/ml or an optical density measured under defined conditions in an ELISA test) which is suited to separate normal healthy samples from diseased samples. e.g. all samples giving values above the cut-off value are considered to be dysplastic, whereas the samples giving values below the cut-off value are considered to be healthy.

In certain embodiments, the threshold or cut-off may be set in a way to separate high grade neoplastic disorders (HSIL or neoplastic disorders corresponding e.g. to invasive carcinoma, high grade dysplasia or histologically assessed CIN 3 lesions) from all less severe stages of neoplastic disorders (e.g. LSIL). In other embodiments, the cut-off may be defined to differentiate healthy samples (NILM) from neoplastic disoders including precursory stages (LSIL and HSIL). It is thus possible to tailor the testing format in order to fit different tasks such as early detection of lesions and even precursors of the lesions or detection of lesions that deserve immediate therapy.

Regarding the detection on a protein level, it is possible to use e.g. antibodies which are directed against said marker molecules. These antibodies can be used in the most varying methods such as Western blot, ELISA or immunoprecipitation. It may be favorable for the antibodies to be fixed on solid carriers such as ELISA plates, reaction vessels, beads, spheres, membranes, colloids such as colloidal metals (e.g. gold), porous members, surfaces of capillaries (e.g. in flow through test), test strips or latex particles. Regarding detection on the nucleic acid level methods such as nucleic acid amplification techniques or hybridization techniques may be applied.

In certain embodiments of the present invention, the detection of the marker molecules is performed from a solution of solubilized body samples. Therefore detection may be carried out in solution or using reagents fixed to a solid phase.

A solid phase as used in the context of the present invention may comprise various embodiments of solid substances such as planar surfaces, particles (including micro-, nano-particles or even smaller particles). In certain embodiments, particles may be provided as spheres, beads, colloids, or the like.

The fixation of reagents to the solid phase may be carried out via direct fixation or via indirect fixation. Direct fixation may be carried out by covalent binding, non-covalent binding, association, or adsorption to surfaces. Indirect fixation may be carried out through binding of the antibody to agents which themselves are directly fixed to solid phases. Binding agents, for example, include avidin, streptavidin, biotin, digioxingenin, antibodies or the like.

The detection of one or more molecular markers may be performed in a single reaction mixture or in two or more separate reaction mixtures. The detection reactions for several marker molecules may for example be performed simultaneously in multi-well reaction vessels. The detection reaction for marker molecules may comprise one or more further reactions with detecting agents either recognizing the initial marker molecules or preferably recognizing the prior molecules (e.g. primary antibodies) used to recognize the initial markers. The detection reaction further may comprise a reporter reaction indicating the level of the markers characteristic for cell proliferative disorders or the normalization markers.

The detection reaction for detecting the level of marker molecules in solubilized samples may be carried out in solution or with reagents fixed to solid phases. In certain embodiments, the detection reaction may be carried out in solution; such procedures may comprise any methods suited for the detection of molecular interactions (binding of an antibody or similar binding agent to an antigen) in solution. The methods for determination of molecular interaction (change in conductivity, mass changes, light-, UV-, IR-, magnetic spectrometric changes, plasmon resonance, etc.) are known to those of skill in the art. In certain embodiments the detection may comprise a method where a complex of detection reagent bound to antigen is adsorbed to a solid phase for detection purpose. Thus, non-covalent bonding of the analytes to solid phases in the course of the detection reaction or even before starting the detection reaction may be used in a method according to the present invention.

In certain embodiments of the invention, immunochemical testing may be performed using a testing device for clinical laboratories. Such testing device may comprise any device suitable for immunochemical testing including any format such as point of care testing devices as well as bench top or laboratory devices. The devices may be e.g. provided as open or closed platform systems. The system may be based on any suitable methodology such as microtiter plates, multiwell plates, flow through or lateral flow systems, microchip or array based systems or bead or membrane based systems. The detection methods employed may comprise any methods known to those of skill in the art useful for immunochemical or nucleic acids based detection reactions. Such detection systems may be e.g. luminescence systems (electroluminescence, bioluminescence, photoluminescence, radioluminescence, chemiluminescence, electrochemoluminescence), fluorescence based systems, conductivity based detection systems, radiation (light, UV, X-ray, gamma etc.), plasmon resonance (e.g. Surface Plasmon Resonance SPR), FRET or any other known method.

The term porous member as used herein shall generally apply to any three dimensional arrangements of porous substances. Such porous member may e.g. comprise compounds as membranes, beads or other.

By means of the present invention it is possible to diagnose cancers early, i.e. in their preliminary stages.

Detection of modified states of polypeptides may for example comprise binding agents specifically recognizing modified or unmodified states of polypeptides. Alternatively enzymes such as phosphatases or glycosylases may be used to remove modifications in molecules. The presence or absence of modifications can thus be detected by determination of mass or charge of the molecules by means of electrophoresis, chromatography, mass spectrometry etc. prior and subsequent to the incubation with a respective enzyme.

In a further embodiment of the present invention, the detection of a series of marker molecules is carried out on the level of polypeptides and simultaneously the detection of a further series of marker molecules and/or of all or some of the same marker molecules is carried out on the level of nucleic acids.

Marker molecules characteristic for medically relevant conditions may e.g. be molecules which influence and/or reflect the proliferation and/or differentiation characteristics of cells and/or tissues. Such molecules may comprise for example cell cycle regulatory proteins, proteins associated with the DNA replication, transmembrane proteins, receptor proteins, signal transducing proteins, calcium binding proteins, proteins containing DNA-binding domains, metalloproteinases, kinases, kinase inhibitors, chaperones, embryogenesis proteins, heat shock proteins or enzymes which modify other proteins posttranslationally thus regulating their activity, or nucleic acids coding for the named proteins. Also mRNA coding for the named proteins may be marker molecules useful according to the present invention. In one embodiment the marker associated with the cell proliferative disorder may be for example uniquely expressed in cells affected by the disorder, may be not expressed in said cells or may be overexpressed in said cells.

The marker molecules for use in a method according to the present invention comprise one or more marker molecules (proteins as well as nucleic acids) chosen from cell cycle regulatory proteins cyclin E, cyclin-dependent kinase inhibitor p16^{INK4a} tumor associated antigens MCM5, MCM6, CDC6, claudin, her2/neu, HPV associated markers, derived from HPV genes L1, L2, E1, E2, E4, E5, E6 or E7, In certain embodiments marker molecules detected according to the method disclosed herein may comprise genes engaged in the DNA replication such as e.g. proteins of the pre-initiation complex or of the replication fork. Such molecules comprise MCM proteins [MCM2 and MCM5] kinases CDCG engaged in the replication process, proteins engaged in the processive replication fork, i.e. PCNA, molecules necessary for the maintenance of cell proliferation, i.e. Ki67 or Ki-S1. Generally the method for development of kits and in-vitro diagnostic devices disclosed herein is suited for kits and in-vitro diagnostic devices based on various marker molecules characteristic for medically relevant conditions. In one embodiment the marker molecules for a medically relevant condition may be a marker for tumors (tumor markers). The marker molecules characteristic for tumors may e.g. be proteins, that are expressed in a non-wild type manner in tumors compared to normal control tissue. Non-wild type expression as used herein may comprise increased or decreased levels of expression, or lack of expression, or expression of non-wild type forms of the respective molecules. Expression of non-wild type forms of a protein may comprise expression of mutated forms of proteins, arising by insertion, deletion, substitution, or frameshift mutations or any other known types of mutations in proteins or nucleic acids. In all cases of the expression of non-wild type proteins or non-wild type levels of proteins the proteins, polypeptides or fragments thereof, or nucleic acids encoding these proteins or polypeptides or fragments of these nucleic acids may be used as molecular markers associated with tumors and may thus be understood under the term "tumor marker" as used in the context of the present invention. Proteins that show non-wild type expression in association with tumors are disclosed for example in the documents WO9904265A2, W00149716A2, W00055633A2 and WO0142792A2.

Cyclin-dependent inhibitors for use in certain embodiments the present invention comprise the cyclin-dependent kinase inhibitors p16^{INK4a}. Beside cyclin-dependent kinase inhibitors the cell cycle regulatory protein p14^{ARF} encoded by an alternative reading frame of the p16^{INK4a} gene may also be used for a method as disclosed herein. For convenience, within the context of the present invention the cell cycle regulatory protein p14^{ARF}, which is by function not a cyclin-dependent kinase inhibitor, shall be included in the expression "cyclin-dependent kinase inhibitor".

"p16" or "cyclin-dependent kinase inhibitor p16^{INK4a}" as used herein refers to cyclin-dependent kinase inhibitor p16INK4a (also denominated as CDKN2 or MTS1) the gene of which is located in chromosomal region 9p21. p16^{INK4a} was first described in Serrano, M., et al., Nature, 1993 Dec 16; 366(6456):704-7. The terms "p16^{INK4a}" or "cyclin-dependent kinase inhibitor p16^{INK4a}" in all their grammatical forms as used in the context of the present invention refers to nucleic acid as well as polypeptide molecules. "p16^{INK4a}" or "cyclin-dependent kinase inhibitor p16^{INK4a}" thus comprises e.g. RNA (mRNA, hnRNA, etc.), DNA (cDNA, genomic DNA, etc.), proteins, polypeptides, proteoglycans, glycoproteins and the respective fragments of these molecules.

In one embodiment of the invention, the marker characteristic for the medically relevant condition may be a cell cycle regulatory protein such as for example a cyclin, a cyclin-dependent kinase or a cyclin-dependent kinase inhibitor. In a further embodiment of the invention the marker characteristic for the medically relevant condition may be a marker associated with a transient or a persistent viral infection. The viral infection may comprise an infection by a human papilloma virus (HPV) such as high risk or low risk HPV. The high risk HPV may comprise HPV subtypes such as e.g. HPV 16, 18, 31, 33, 35, 39, 45, 51, 52, 56 and 58. The markers for HPV infection may e.g. comprise HPV expression products of HPV genes L1, L2, E2, E4, E5, E6 or E7. In a third embodiment of the invention a marker characteristic for a viral infection may be used in combination with any other marker for a medically relevant condition such as e.g. in combination with a cell cycle regulatory protein. Combinations of marker molecules, which may be of special interest with respect to HPV association are e.g. disclosed in W00208764.

In one embodiment, cell cycle regulatory proteins for use in combination with HPV markers may for example be chosen from a group comprising pRb, p53, p14^{ARF}, cyclin-dependent kinase inhibitors. In one special embodiment for example p16^{INK4a} may be used in combination with markers for HPV infection (e.g. L1, L2, E2, E4, E5, E6 or E7).

For the method of detection of medically relevant conditions as disclosed herein principally any marker molecules may be applied for several medically relevant conditions. However certain marker molecules are known to be associated with specific medically relevant conditions. Those of skill in the art know which marker molecules could reasonably be used in a method according to the present invention for the detection of a medically relevant condition in a solubilized body sample. Below examples of medically relevant conditions and marker molecules suitable for application in a method as described herein are given. In Table 3 below examples of medically relevant conditions and marker molecules suitable for application in a method according to the present invention are given. The information is intended to exemplify the method as disclosed herein and not to restrict the scope of the invention, which is defined by the appended claims.

**Table 3**

| **Medically relevant condition** | **Examples of suitable Marker Molecules for a method as disclosed herein** | |
|---|---|---|
| | **Protein level** | **Nucleic Acid Level** |
| Cervical Carcinoma | p16^{INK4a}, p14^{ARF}, claudin, p19^{INK4d}, Ki67, Cyclin E, Cyclin D, MCM-5, MCM-2, HPV E7, HPV E2, HPV E4, HPV L1, CK18, CD-46, NMP-173, Brn-3, Mn-antigen; | p16^{INK4a}, p14^{ARF}, claudin, p19^{INK4d}, Ki67, Cyclin E, Cyclin D, MCM-5, MCM-2, HPV E7, HPV E6, HPV E2, HPV E4, HPV L1, (any HPV nucleic acid sequence specially hrHPV), NMP-173, Bm-3, Mn-antigen, TSLC-1, PTEN, |
| Bladder Cancer | survivin, MCM-5, MCM-2, CDC-6, Her-2/Neu, MMP-2, Cyclin-E, KIAA1096, p21^{WAF1/CIP1}, pRB, MDM2, NMP-22 | NY-ES01, MCM-5, MCM-2, CDC-6, p53, Her-2/Neu, MMP-2, Cyclin-E, KIAA1096, p21^{WAF1/CIP1}, pRB, MDM2, NMP-22 |
| Colorectal Cancer | Claudin, DNAseX, MCM-5, MCM-2, caveolin-1. cathepsin-B, Cyclin D1, Cyclin E, c-myc, TGF-beta, Her-2/Neu | Claudin, DNAseX, MCM-5, MCM-2, caveolin-1. cathepsin-B, Cyclin D1, Cyclin E, c-myc, TGF-beta, Her-2/Neu |
| Small Cell Lung Cancer | p16^{INK4a}, GRP, Her-2/Neu, cyclo-oxigenase-2, NSE, CA 15-3 | p16^{INK4a}, GRP, Her-2/Neu, cyclo-oxigenase-2, NSE, CA 15-3 |
| Non Small Cell Lung Cancer | NSE, GRP, Cyclin D1, Her-2/Neu, SCC, CEA, CA 19-9 | NSE, GRP, Cyclin D1, Her-2/Neu, SCC, CEA, CA 19-9 |
| Breast Cancer | Cyclin D3, Her-2/Neu, e-cadherin, survivin, cathepsin D | BRCA2, Cyclin D3, Her-2INeu, e-cadherin, survivin, cathepsin D |
| Inflammation ion various cytological specimens | Leukocyte specific proteins; Granulocyte specific proteins | Leukocyte specific nucleic acids; Granulocyte specific nucleic acids |

The marker molecules for medically relevant conditions according to the present invention may be characteristic for the presence of absence of a medically relevant condition. In one embodiment of the present invention the marker molecules may be characteristic for specific properties of medically relevant conditions. Such characteristics may comprise the progression potential, prognostic information, behavior respective certain therapeutic treatments of the medically relevant condition. The marker molecules characteristic for medically relevant conditions may therefore be marker molecules useful for the determination of prognosis of individuals affected by medically relevant conditions or for stratification of therapy of individuals affected by medically relevant conditions. This may in certain embodiments apply to the determination of presence or absence of the expression of certain marker molecules that are indicative of positive or negative prognosis in specific medically relevant conditions (e.g. expression Level of p16, Her-2/Neu, Claudin or others in breast cancer etc.) Examples of marker molecules that allow to assess prognosis of individuals affected with specific medically relevant conditions are known to those of skill in the art. Any such markers known from cell based cytological or histological procedures may be used in a method according to the present invention. The assessment of prognosis may e.g. be performed during or after primary diagnosis of the medically relevant condition, during or after (surgical) treatment of the medically relevant condition or at any other stage of the history of the respective medically relevant condition. In other embodiments the method according to the present invention may be used for the stratification of treatment of individuals affected with medically relevant conditions. Such stratification may e.g. comprise the selection of certain therapeutic compounds in the sense of Theragnostic procedures (as used e.g. for selection of patients for therapy with Herceptin or the like), selection of patients for chemotherapy, for radiotherapy or for any other decision on the further therapeutic treatment of individuals or generally for the decision on the medical treatment of individuals such as monitoring follow up or the like.

In certain embodiments of the present invention the marker molecules characteristic for medically relevant conditions may be markers for indicative of progression of the medically relevant condition. In certain further embodiments of the present invention the marker molecules characteristic for the progression of medically relevant conditions may be used in combination with marker molecules characteristic for the mere presence of said medically relevant condition.

The application describes a method for development of kits and in-vitro diagnostic devices for diagnosis of medically relevant conditions from solubilized body samples, wherein the development is performed using body samples provided as preserved cells in a cell-preservation medium and wherein the preserved cells are intended and prepared for use in cytological examination processes such as Liquid Based Cytology processes (not part of the claimed invention). The samples intended for Liquid Based Cytology processes (in the following denominated as LBC samples) are solubilized in an appropriate lysis medium and are used for development activities of kits and in-vitro diagnostic devices for detection of medically relevant conditions from solubilized body samples on the basis of biochemical non-cell-based analysis.

The method of development of kits and in-vitro diagnostic devices is applicable to kits and in-vitro diagnostic devices for the detection and diagnosis of all kinds of medically relevant conditions.

Development as used in the context of the present application shall pertain to all design and development activities performed for enabling a manufacturer for controlled production of a finished kit or in-vitro diagnostic device intended for commercial distribution or sale of said kit or in-vitro diagnostic device. Development of kits and in-vitro diagnostic devices as used in the context of the present invention accordingly shall pertain to all activities in connection with the design and development, design- and development-verification, design- and development-validation, assessment of performance data, assessment of safety and effectiveness data of kits and in-vitro diagnostic devices. In one embodiment development shall pertain to the testing of design- and development-outputs of kits and in-vitro diagnostic devices for suitability regarding the proposed intended use. Intended use in this respect shall be understood as the detection or diagnostic purposes for which the kit or in-vitro diagnostic device shall be applied.

The method for development of kits and in-vitro diagnostic devices described herein is directed to development of kits and in-vitro diagnostic devices for biochemical testing formats. In these testing formats the presence or absence and/or the level of marker molecules in solubilized body samples is detected. Solubilization of the body samples is performed using a suitable lysis medium as detailed above. The development of a kit or in-vitro diagnostic device according to the present invention makes use of LBC samples for design, development, design and development verification, design and development validation. Furthermore a method for development of kits and in-vitro diagnostic devices on the basis of LBC samples as disclosed herein is any method that employs LBC samples for provision of technical documentation and/or of evidence for safety and effectiveness for the purpose of regulatory clearance or approval of the respective kit or in-vitro diagnostic device before the regulatory authorities and regulatory (notified) bodies if applicable. The method of development of kits and in-vitro diagnostic devices as disclosed herein may employ LBC samples in all stages of the design, development, verification, validation, provision of data for regulatory submission and clearance / approval, or may employ LBC samples only in one or some of the named steps of kit or in-vitro diagnostic device design and development. In one embodiment the method of development of the kits or in-vitro diagnostic devices is a method for design and development of said kits and in-vitro diagnostic devices, wherein the LBC samples are used for design and development verification and/or validation. In another embodiment of the invention the method of development of kits and in-vitro diagnostic devices is a method for provision of data for regulatory submission and clearance / approval of kits and/or in-vitro diagnostic devices before national or regional regulatory authorities and/ or national or regional regulatory (notified) bodies, wherein LBC samples are used for the provision of technical data, performance data or safety and effectiveness data regarding the kit or in-vitro diagnostic device. In a further embodiment of the invention the method of development of kits and in-vitro diagnostic devices is a method where the latter methods are combined.

The kits and in-vitro diagnostic devices used in the method of the present invention are characterized in that the detection of the marker molecules characteristic for medically relevant conditions is performed on the basis of biochemical non-cell-based analysis. Biochemical non-cell-based analysis as used in the context of the present invention shall refer to all methods where an analyte or a marker molecule is detected in a solution, wherein no information on cellular morphology or on tissue architecture is used for assessment of diagnosis. (Cells and tissue remnants need not necessarily be absent from such solution). Said biochemical non-cell-based analysis is founded on information obtained from the detection of the presence or absence of one or more marker molecules in the solution under investigation or from the detection of the levels of one or more marker molecules in the solution under investigation. In certain embodiments of the present invention the kits and in-vitro diagnostic devices are designed to detect only one single marker molecule. In further embodiments of the present invention the kits and in-vitro diagnostic devices are designed to detect a set of marker molecules. Generally the method of development as disclosed herein may be applied to several types of kit and in-vitro diagnostic devices.

Kits as used in the context of the present invention are compositions of components provided for performance of an analytical testing procedure. The kit may comprise all or some of the reagents and materials necessary for proper performance of the test. Furthermore the kit may in certain embodiments of the invention comprise instructions for an appropriate application of the kit components including e.g. an exemplary testing protocol, warnings and hazard information and further accessory information for the user of the kit.

The kits used in the methods of the present invention may e.g. comprise:
(a) a reagent for detecting the expression of a marker molecule, e.g. a probe directed against a protein and parts thereof, respectively,
(b) a lysis medium for solubilization of a body sample,
(c) conventional auxiliary agents, such as buffers, carriers, markers, etc., and optionally
(d) an agent for control reactions, e.g. a marker molecule protein and parts thereof, respectively, or a preparation of cells.

Furthermore, one or several of the individual components may be present. For example, the detection reagent and other reagents fixed to a solid phase may be present. In one embodiment the kit comprises a reagent for detection of a marker molecule fixed to solid phases and no detection reagents of other specificities fixed to solid phases.

In certain embodiments the kits for detection of marker molecules inhibitors are provided as in-vitro diagnostic devices.

In often embodiments the kit may be provided as in-vitro diagnostic device An in-vitro diagnostic device is a kit as defined above, that is intended for assessment of diagnosis of a medically relevant condition from human or animal body samples.

An in-vitro diagnostic device is a kit as defined above, that is intended for assessment of diagnosis of a medically relevant condition from human or animal body samples. In certain embodiments of the invention an in-vitro diagnostic device shall be any device that falls in the scope of the definition of in-vitro diagnostic medical device as given in the directive 98/79 EC under Article 1 (b):
'*in vitro diagnostic medical device' means any medical device which is a reagent product, calibrator, control material, kit, instrument, apparatus, equipment, or system, whether used alone or in combination, intended by the manufacturer to be used in vitro for the examination of specimens, including blood and tissue donations, derived from the human body, solely or principally for the purpose of providing information concerning a physiological or pathological state; or concerning a congenital abnormality; or to determine the safety and compatibility with potential recipients ; or to monitor therapeutic measures*.

In vitro diagnostic device shall also apply to U.S. Class I IVD and generally to in-vitro diagnostic devices that are provided without Claims regarding their diagnostic performance. Therefore also any kind of ASR or the like shall be understood to be an in-vitro diagnostic device as used herein. In one embodiment of the present invention the in-vitro diagnostic device is characterized by solid phase fixed detection reagents specific for a marker molecules.

In in-vitro diagnostic device may comprise probes directed against marker molecules fixed to a solid phase allowing assessment of diagnosis of medically relevant conditions in a solubilized sample. The probes may e.g. comprise antibodies or fragments thereof directed against marker moelcules. It is an advantage of in-vitro diagnostic devices to allow for easy and economic assessment of diagnosis of medically relevant conditions. The test may be suited for screening purposes as well as for diagnostic purposes and may be applied in primary diagnosis as well as in monitoring of disease course. The in-vitro diagnostic devices may in certain embodiments be applicable for use in clinical laboratories, for point of care testing or even for self testing.

The in-vitro diagnostic devices comprising solid phase fixed reagents for the detection marker moelcules may be useful for the detection of various different medically relevant conditions. The in-vitro diagnostic devices may be applied for analysis of any kind of lysed body samples.

The probes can be fixed to the solid phase via direct fixation or via indirect fixation. Direct fixation can be done by covalent or non-covalent binding or association to surfaces. Indirect fixation can be done through binding of the antibody to agents which themselves are directly fixed to solid phases. Such agents may comprise antibodies or other binding agents like avidin, streptavidin, biotin, digioxingenin or the like.

The in-vitro diagnostic devices useful for the present invention are selected from the group consisting of
a. an ELISA device comprising antibodies directed against marker molecules fixed to ELISA plates, ELISA stripes or ELISA wells;
b. a lateral flow test device, comprising antibodies directed against marker molecules fixed to test strips, colloidal gold particles or latex particles;
c. a flow through assay device, comprising antibodies directed against marker molecules fixed to a porous member, or to the surface of capillaries;
d. a latex agglutination assay device, comprising antibodies directed against marker molecules fixed to latex particles; and
e. an immunoassay device, comprising antibodies directed against marker molecules fixed to beads, membranes, or microspheres.

The ELISA devices may be of any kind known to those of skill in the art. These devices comprise devices for sandwich ELISA formats, for competitive ELISA formats and any other ELISA formats.

In one embodiment, the in-vitro diagnostic device may comprise a lysis medium for solubilization of the sample. In a further embodiment the in-vitro diagnostic device comprises reagents for detection of one specific marker molecule fixed to solid phases and no detection reagents of other specificities fixed to solid phases.

Lateral flow assay devices for use as an in-vitro diagnostic device are any lateral flow assay devices employing at least one reagent binding to marker molecule fixed to a solid phase. Such devices may employ various mechanisms for visualization of the test result. In certain embodiments, the tests may employ secondary detection reagents directed against marker molecules or another components participating in the test coupled to detectable moieties. The detectable moieties may comprise colloidal gold, (colored) latex particles and others.

Flow through assay devices for use in the present invention may comprise devices based on capillaries or on porous members (such as membranes, beads or other three dimensional arrangements of porous substances). Depending on the embodiment the size of pores or capillaries need to adjusted to ensure optimal flow conditions.

The kits and in-vitro diagnostic devices can be intended for use with any kind of solubilized body sample. In the present invention the diagnostic method disclosed herein is intended for use with LBC samples only. In this case the kit or in-vitro diagnostic device can be developed and manufactured for analysis of solubilized LBC samples as an adjunctive or conjunctive test to the cytological analysis or as a stand alone test.

Applicable formats for the detection reactions applied in the kits and in-vitro diagnostic devices according to the present invention may be blotting techniques, such as Westem-Blot, Southern-blot, Northern-blot. The blotting techniques are known to those of ordinary skill in the art and may be performed for example as electro-blots, semidry-blots, vacuum-blots or dot-blots. Furthermore immunological methods for detection of molecules may be applied, such as for example immunoprecipitation or immunological assays, such as EIA, ELISA, RIA, lateral flow assays, flow through assays, immunochromatographic strips, etc. Immunoassays for use in the invention may comprise competitive as well as non-competitive immunoassays.

The testing procedures for which the kits and in-vitro diagnostic devices shall be applied according to the present invention include detecting the levels of marker molecules characteristic for medically relevant conditions in the test sample on the basis of biochemical non-cell-based analysis. The markers suitable for these testing procedures according to the present invention may be of various origin. The expression pattern of a marker, that is suitable for the detection of medically relevant conditions in question, may be dependent on the proliferative status of cells, on the differentiation status, on the cell type or on the organism.

The method for development of kits and in-vitro diagnostic devices as detailed herein makes use of LBC samples in any way that is suitable for gathering data on the performance characteristics of the kit or device under development. Generally LBC samples are used as a source of body samples to be used in the course of the kit or in-vitro diagnostic device development. In one embodiment of the present invention the LBC sample is supplied as a left over specimen, wherein a cytological specimen has been prepared from the LBC sample before, during or after use of parts of the LBC sample for the development method according to the present invention. In another embodiment the LBC sample has been obtained solely for the purpose of use in a method of development according to the present invention. In this case a second and may be third LBC sample or even sample prepared by conventional non thin-layer methods for cytological evaluation may have been obtained before or after the sampling of the respective LBC sample used in the method of development according to the present invention.

In another embodiment of the present invention the body sample may be further purified before being lysed. Such purification procedures may for example comprise washing away of contaminants such as mucus or the like, separation or concentration of cellular components, preserving and transporting of the cells.In one embodiment for example the cells may be separated by means of flow cytometry or other suitable forms of cell sorting known to those of skill in the art. Thus the cellular components of the raw samples are included in a single sample solution.

The invention is illustrated further by the following examples, which are not to be construed as limiting the invention in scope to the specific procedures described in them.

### EXAMPLES

### Example 1 (Reference example) : Detection of Cervical Intraepithelial Neoplasia in an ELISA test format.

33 cervical swabs provided in a lysis medium were subjected to ELISA based detection of overexpression of cyclin-dependent kinase inhibitor p16^{INK4a} in solutions prepared from the cells contained in the swabs. The ELISA testing was performed as follows:

### (A) Cell Lysis

Cervical swab brushes were given into 15 ml vessels, containing 2 ml of mtm lysis medium (2% Triton X-100, 0.4% SDS, 0.6mM PMSF in PBS). Cervical cells present in the brush were lysed for at least 20h. The lysates of the cervical swab samples were then transferred in 2 ml tubes and were centrifuged at 4°C (15 min at 28.000 x g (16.600rpm Highspeed Centrifuge JEC Multi RF)); Supernatant was transferred to a fresh tube. The supernatant may be stored at -20 °C.

### (B) Performing the ELISA

### Coating of ELISA-plates

Stock-solution of p16^{INK4a} specific antibody clone mtm E6H4 was diluted in PBS to give ready-to-use coating solution.

50µl of the coating solution was added to each well of the ELISA plates.

For coating, the plates were incubated overnight at 4°C.

Coating solution was removed from the ELISA plates and the plates were rinsed using an automated ELISA washer as follows:
- 7 x 250µl washing buffer (0.1% Tween20 (v/v) in PBS)
after removing remnants of the washing buffer, 300µl blocking buffer (2% BSA in PBS) was added to each well. Plates were incubated for 1h on a rocking device at ambient temperature.

### Incubation with samples

After removing the blocking buffer, 100µl of the lysed cell sample was added to each well. Lysates of HeLa-cells were used as positive control;

For purpose of calibration of the test, different concentrations of recombinant p16^{INK4a} protein (0 pg/ml, 50 pg/ml, 100 pg/ml, 200 pg/ml, 400 pg/ml, 800 pg/ml) were included in the test.

Samples were incubated for 1 h at room temperature.

Thereafter washing was performed on an automated ELISA washer as follows:
- 7 x 250µl washing buffer. The remaining buffer was removed.

### Incubation with detection antibody

Working solution of biotinylated secondary antibody clone mtm D7D7 specific for p16^{INK4a} protein was prepared by dilution of stock solution.

100µl of working solution was added to each well. After incubation for 1h at RT, antibody solution was removed and ELISA plates were washed by an automated ELISA washer
- 7 x with 250µl washing buffer.

### Detection

Streptavidin-HRP-polymers (1mg/ml) were pre-diluted 1:10. (4µl +36µl incubation buffer); Final incubation solution was prepared by dilution 1:300 in incubation buffer (0,1% BSA in PBS) to a final concentration of 0,33 µg/ml.

µl of this solution were added to each well and incubated for 1 h at RT.

Thereafter, the buffer was removed and the plates were washed manually with 200 µl washing buffer per well 5 times.

### Substrate incubation

TMB-substrate was equilibrated to 25°C for 1h in the dark.

100µl of substrate solution was added to each well.

The ELISA plates were incubated at 25°C for exactly 15 min in the dark. Then the reaction was stopped by addition of 80 µl 2,5M H2SO4.

Within 5 min. after stopping the reaction, OD 450 nm was determined. After evaluation of the results, each sample returned a value for the OD.

Results of this experiment are given in Table 4. The ELISA results were compared to the diagnostic results of a Papanicolaou test (PAP test, cervical cytology) from the same patients. The cervical cytology were evaluated according to the Munich Classification II (1990). Pap II encompasses benign cells, cervicitis and metaplasia, Pap IV encompasses severe dysplasia and carcinoma in situ. It turned out that samples returning an OD greater than 0.9 in the ELISA correspond to samples, that are classified as dysplastic by the conventional cytological PAP test.
Applying OD 0.9 as threshold for the evaluation of the samples, the ELISA results may be reported as follows:

**Table 4**

| **Diagnosis/ELISA results** | **ELISA positive** | **ELISA negative** |
|---|---|---|
| Pap II | 0 | 30 |
| Pap IV | 3 | 0 |

The ELISA test is positive in all samples (100%) from women having severe dysplasia and is negative in all 30 samples (100%) of women having no dysplasia.

Using the threshold evaluated in these experiments, cytological specimens of 300 patients were tested in the presented ELISA testing format. In this experiments the specimens identified as being dysplastic by cytological examination may also be identified as dysplastic in the ELISA testing format.

The results show, that the quantification of p16^{INK4a} protein in solubilized patient samples allows to detect dysplasias from the samples. The diagnosis in the present example is based on the comparison of the level of p16^{INK4a} determined in a specific patient sample to the level known to be present in normal non-dysplastic samples. The comparison is carried out in the testing format by applying a threshold value for the OD determined in the ELISA above which the sample is to be classified as positive.

### Example 2: Detection of Cervical Intraepithelial Neoplasia in an Lateral Flow test format.

Nine cervical swabs provided in PreservCyt (Cytyc Corporation, Boxborough, MA) solution have been subjected to conventional PAP testing and simultaneously to lateral flow based detection of overexpression of cyclin-dependent kinase inhibitor p16^{INK4a} in solutions prepared from the cell suspensions obtained from the swabs. The lateral flow testing was performed as follows:

### (A) Cell Lysis

10 ml of the cell suspensions from the individual cervical swab samples provided as PreservCyt™ fixed materials were transferred to a 15 ml reaction vessel. The samples were centrifuged 15 min at ambient temperature at 1500 x g (3000rpm, Heraeus Varifuge, rotor 8074); supernatant was discarded, and remaining methanol allowed to evaporate (15 min at ambient temperature); the pellet was solubilized in 500µl Lysisbuffer and transferred to a 1.5 ml reaction vessel. The solution was centrifuged at 4°C (15 min at 28000 x g (16600rpm Microcentrifuge Biofuge fresco)); Supernatant was transferred to a fresh tube. Supernatant may be stored at -20°C.

### (B)Performing the Lateral Flow Assay

### Applying capture antibody to membrane

Stock solution of p16^{INK4a} specific antibody clone mtm E6H4 was diluted in TBS (containing 1% bovine serum albumin) to give ready-to-use spotting solution with a final concentration of 1 mg antibody/ml. The ready-to-use solution was spotted onto nitrocellulose membrane at 30 µl/30 cm. Whatman wicks were attached to one end of the nitrocellulose and dipsticks are dried for 1 hour at 37°C. Then they were allowed to equilibrate at room temperature and cut into 4mm width dipsticks.

### Preparation of Conjugate solution

Stock-solution of p16^{INK4a} specific antibody clone mtm D7D7, conjugated to colloidal gold (40 nm particle size) was diluted in TBS (containing 1% bovine serum albumin) to give ready-to-use detection antibody solution with a final concentration of 1.0 OD at 520nm.

### Incubation with samples

Then 20 µl of the lysed cell samples were added to 20 µl ready-to-use detection antibody solution in a microtiter well and mixed. Dipstick, coated with capture antibody clone E6H4 was added to the well, sample was soaked and run to completion. The signal was read while the dipstick is still wet.

### Results

In our testing format, 2 samples (samples 1 and 2) classified as PAP IVa by PAP staining and therefore containing dysplastic cells, gave clearly visible purple bands in the area of spotted capture antibody. In contrast, no band was detected for the other 7 samples (samples 3-9), classified as PAP II-III by PAP staining and therefore not containing dysplastic cells.
ELISA was performed by the same protocols given in Example 1. The results are shown in Table 5.

**Table 5**

| **Sample** | **Diagnosis** | **ELISA OD** |
|---|---|---|
| 1 | Pap IVa | 2.209 |
| 2 | PAP IVa | 0.536 |
| 3 | PAP III | 0.067 |
| 4 | PAP II | 0.113 |
| 5 | PAP II | 0.095 |
| 6 | PAP II | 0.284 |
| 7 | PAP II | 0.192 |
| 8 | PAP II | 0.138 |
| 9 | PAP II | 0.07 |

The invention, and the manner and process of making and using it, are now described in such, full, clear, concise and exact terms as to enable any person skilled in the art to which it pertains, to make and use the same. It is to be understood that the foregoing describes preferred embodiments of the present invention and that modifications may be made therein without departing from the scope of the present invention as set forth in the claims. To particularly point and distinctly claim the subject matter regarded as invention, the following claims conclude this specification.

### Example 3: Detection of p16^{INK4a} and p14^{ARF} transcripts by RT-PCR (not part of the claimed invention)

Cervical samples from 50 individuals were used for this analysis. For each individual two samples were obtained, one in Universal Collection Medium and one in PreservCyt™ solution. Both samples were obtained during the same examination session. For each of the individuals a diagnosis based on analysis of a cervical thin layer specimen prepared out of the PreservCyt™ solution was available. 20 of the samples included in the present study were chosen to be diagnosed as NILM, 20 samples were chosen to be LSIL and 10 samples were chosen to be HSIL. From all samples the level of transcripts of p16^{INK4a} and of p14^{ARF} have been determined on an mRNA level by RT-PCR according to the following protocol: For performance of the analysis the cells were pelleted from the UCM and PreservCyt™ solutions by centrifugation. The pellets obtained were directly subjected to the RNA preparation procedure.
The pellet was diluted and resuspended in ready to use RLT Buffer. After adding 70% Ethanol to the homogenised lysat the suspension was mixed by pipetting .
Purification and isolation of RNA was performed using QIAamp Spin-columns according to the directions of the manufacturer.

RNA concentrationwas determined photometrically at 260nm. For reverse transcriptase reaction from 100ng up to 500ng RNA were used. DNA was degraded by DNase reaction as follows

| | |
|---|---|
| 17,0 µL | RNA (6-30ng/µL) |
| 1,0 µl | DNAse I Amp Grade (1 Unit/µl)(Invitrogen) |
| 2,0 µl | DNAse Reaction Buffer (10x)(Invitrogen) |
| 20, 0µl | total volume |

Incubation was performed for 15 Min at 25°C and the reaction was stopped by adding 2pl EDTA 25 mM and incubation for 10 Min at 65°C.
cDNA synthesis was performed using the whole volume of the DNase digest using Omniscript reverse transcriptase in the presence of RNAsin.
The reaction was performed for 2 h at 37°C and subsequently 5 Min at 93°C. Afterwards the mixture was stored at 4°C.
For use in RT-PCR the 40µl cDNA reaction mixture was diluted with 30µl RNase-free water to a volume of 70 µl. This renders a ready to use cDNA solution for the Taqman-PCR with an approximate cDNA concentration of about 7-36 ng/5µl.The primers used were:

| | |
|---|---|
| Primer p16^{INK4a}, forward: | 5'-CGA ATA GTT ACG GTC GGA GG-3' |
| Primer p16^{INK4a}, reverse. | 5'-ACC AGC GTG TCC AGG AAG-3' |
| | |
| Primer p14^{ARF}, forward: | 5'-CCG CCG CGA GTG AGG GTT-3' |
| Primer p14^{ARF'}, reverse. | 5'-TGC CCA TCA TCA TGA CCT GGT CT-3' |

As controls PCR reactions for β-actin and GAPDH were performed using the primers:

| | |
|---|---|
| Primer (63) β-Actin, forward: | 5'-CCT AAA AGC CAC CCC ACT TCT C-3' |
| Primer (64) β-Actin, reverse: | 5'-ATG CTA TCA CCT CCC CTG TGT G-3' |
| Primer GAPDH, forward: | 5'-ACC ACA GTC CAT GCC ATC AC-3' |
| Primer GAPDH, reverse: | 5'-TCC ACC ACC CTG TTG CTG TA-3' |

Each primer was used at a concentration of 300nmol. The reaction mixture for RT-PCR was composed as follows:

| | |
|---|---|
| 12,5 µl | SYBR-Umix |
| 0,25 µl | Primer Mix |
| 7,25 µl | Wasser für die Molekularbiologie |
| 5,0 µl | cDNA solution |
| 25,0 µl | total volume |

The conditions for the 2 Step Real Time PCR are:
1^{st} Step: 50°C 2 Min, 95°C 10 Min
2^{nd} Step: 95°C 15 sec, 60°C 1 Min 10 sec, 40 Cycles
Evaluation of the RT-PCR results was performed by estimation of the degree of overexpression of the p16^{INK4a} transcripts on the basis of the level of transcripts detected in the sample specimens compared to levels of transcripts present in normal tissue or cell specimens. Normalization with respect to the level of housekeeping genes detected in each sample was performed for the levels of p14^{ARF} prior to analysis of overexpression. An overexpression of 0 to 24 times compared to normal tissue was regarded as not relevant. Only levels of overexpression of p16^{INK4a} and p14^{ARF} higher than 24 times were regarded as significantly elevated transcript levels in the samples. It must be noted, that this scheme for evaluation is only one of several equally suitable methods. Those of skill in the art know how the results of RT-PCR may be used to estimate transcript levels and to do correlations to clinical parameters of specimens. The Threshold mentioned in this example is exemplary and may vary depending on conditions.
The values obtained from the UCM specimen of one sample and the corresponding PreservCyt™ specimen gave the same results.
A comparison of the detected transcript level to the diagnosis of the correlated specimen from cytology showed good correlation between elevated transcript levels and presence of cervical lesions diagnosed on the basis of cytological thinlayer specimens.
The correlation was as follows:

**Table 6:**

| | Cytological Diagnosis | | | |
|---|---|---|---|---|
| | **Gene** | **NILM** | **LSIL** | **HSIL** |
| Level of transcripts of p16^{INK4a} as detected in RT-PCR [expressed as times elevation compared to levels found in normal specimens] | | | | |
| 0 to 24 times elevated | p16^{INK4a} | 19 | 0 | - |
| | p14^{ARF} | 16 | 3 | - |
| higher than 24 times elevated | p16^{INK4a} | 1 | 20 | 10 |
| | p14^{ARF} | 4 | 17 | 10 |

The experiment shows that a method for detection of transcript level of p16^{INK4a} and p14^{ARF} in lysed cervical samples is suited for assessment of diagnosis of cervical lesions and their precursors. The tested cell preservation solutions turned out to be equally suitable for the disclosed method. The transcript levels of both tested genes may be used for adding in assessment of diagnosis of cervical intraepithelial neoplasia, wherein p16^{INK4a} shows slightly better results than p14^{ARF}.

### Example 4: Hybrid capture analysis of transcript levels of p16^{INK4a} and p14^{ARF} in Liquid based cytology samples from Urineswabs from the oral cavity, sputum and from cervical swabs. (not part of the claimed invention)

Each 10 LBC samples in PreservCyt™ or CytoLyt™ solution respectively of cervical swabs from individuals with diagnosed HSIL lesion, of sputum from individuals with small cell lung cancer, and of swabs from the oral cavity from individual with cancer of the oral cavity were used for each of the marker molecules in the present example. For the cervical and oral specimens a hybrid capture analysis for the presence of hrHPV types and of transcripts of p16^{INK4a} and p14^{ARF} was performed. Hybrid capture for hrHPV types was performed using HybridCaptrue hc2 test by Digene Corp.. Hybrid capture analysis for the transcripts of the named cyclin-dependent kinase inhibitors was performed as described below.

### (A) Cell Lysis

For the present example the amount of the LBC sample was dependent on the cell content of the LBC sample. A thin-Layer specimen of each sample was prepared using a Cytyc ThinPrep™ processor. The mass of the LKBC sample was determined before and after the preparation of the thin-layer specimen. As the ThinPrep™ processor consumes upon each processing only the amount of sample necessary for a specific cell density on the filter the volume consumed is a measure for the relative cell concentration in the LBC sample. In the present example from each LBC two times the mass consumed for the preparation of the thin-layer specimen by the TninPrep™ processor was applied for the hybrid capture analysis. (Samples for which the cellularity of the LBC sample was too low have been excluded.)
For performance of the analysis the cells were pelleted from the CytoLyt™ and PreservCyt™ solutions by centrifugation. The pellets obtained were directly subjected to the RNA preparation procedure.
The pellet was diluted and resuspended in ready to use RLT Buffer. After adding 70% Ethanol to the homogenised lysat the suspension was mixed by pipetting .
Purification and isolation of RNA was performed using QIAamp Spin-columns according to the directions of the manufacturer.
For detection of the p16^{INK4a} mRNA a mixture of 40-mer DNA oligonucleotide probes specific for p16^{INK4a} and p14^{ARF} were used. The most suitable probes in the mixture had the following sequences: p14^{ARF}:
5'-GCT CCG CCA CTC GGG CGC TGC CCA TCA TCA TGA CCT GGT C-3'
5'-GCC ACT CGG GCG CTG CCC ATC ATC ATG ACC TGG TCT TCT A -3'
5'-TCG GGC GCT GCC CAT CAT CAT GAC CTG GTC TTC TAG GAA G-3'
5'-CGC TGC CCA TCA TCA TGA CCT GGT CTT CTA GGA AGC GGC T -3'
5'-CCC ATC ATC ATG ACC TGG TCT TCT AGG AAG CGG CTG CTG C -3'
5'-CAT CAT CAT GAC CTG GTC TTC TAG GAA GCG GCT GCT GCC CTA G-3'
5'-TGC CCA TCA TCA TGA CCT GGT CTT CTA GGA AG-3'
5'-ATC ATC ATG ACC TGG TCT TCT AGG AAG CGG CTG CTG CCC TAG-3'

It is advantageous to place the probes on the border between Exon 1β and Exon2 of the mRNA to ensure that only p14^{ARF} specific mRNA is recognized by the probes (Note: The situation is similar for specific PCR conditions for p16^{INK4a} and p14^{ARF} respectively; primer pairs could be selected to cover the Exon boundary within the amplificat.). Any other probes specifically recognizing p14^{ARF} mRNA may be used similarly. The probes disclosed in this example are used as an example and are not intended to restrict the scope of the invention. Probe sequence comprising the above sequences or fragments thereof may similarly be used for a method as disclosed herein.

For p16^{INK4a} promising probe sequences are the following:
5'-CTC CGC CAC TCG GGC GCT GCC CAT CAT CAT GAC CTG GAT CGG-3'
5'-ACT CGG GCG CTG CCC ATC ATC ATG ACC TGG ATC GGC CTC-3'
5'-CGG GCG CTG CCC ATC ATC ATG ACC TGG ATC GGC CTC CGA-3'
5'-GCT GCC CAT CAT CAT GAC CTG GAT CGG CCT CCG ACC GTA A-3'
5'-CAT CAT CAT GAC CTG GAT CGG CCT CCG ACC GTA ACT ATT C-3'
5'-ATC ATC ATG ACC TGG ATC GGC CTC CGA CCG TAA CTA TTC GGT GC-3'
5'-AGC AGC TCC GCC ACT CGG GCG CTG CCC ATC ATC ATG ACC TGG ATC-3'
5'-ATC ATC ATG ACC TGG ATC GGC CTC CGA CCG TAA CTA TTC-3'
5'-TCA TCA TGA CCT GGA TCG GCC TCC GAC CGT AAC TAT TCG GT-3'

Similar as to the situation with p14^{ARF} for p16^{INK4a} the probes preferably are places to overlap with the exon boundary of Exon 1 a to Exon 2. This provision could ensure that p16^{INK4a} is recognized and no other mRNA transcribed from the INK4 locus. Further more the comments given for the probes to p14^{ARF} apply here mutandis mutatis.
The labeled probe mixture was added to the total cellular RNA extract. For hybridization the mixture was incubated at 65° C for 30 Min.

### (B) Performing the ELISA

For the detection of the RNA-DNA hybrids microtiter plates coated with anti-RNA/DNA-hybrid antibodies available from Digene Corp. were used. The hybridization solution was added directly to the microtiter plates and incubated for 1h at ambient temperature. The plates are washed according to the instructions by the manufacturer. Detection was performed using the secondary anti-RNA/DNA-hybrid antibody and detection reagents provided by Digene Corp..
The hybrid capture assay revealed positive results for p16^{INK4a} for all cervical specimens. This result was in concordance with all cervical specimens being positive for hr HPV by Hybrid Capture. About half of the cancer specimens from the oral cavity was tested as p16^{INK4a} overexpressing. All of these specimens being positive for p16^{INK4a} have been tested for hrHPV by hc2. There was significant correlation between HPV positivity and p16^{INK4a} overexpression in cancer of the oral cavity. For small cell lung cancer p16 could be detected as positive in 8 out of 10 of the tested cases. All results for p16^{INK4a} obtained by the hybrid capture test could be confirmed by immuno-cytochemical analysis of the thin-layer specimens.
For p14^{ARF} the results for cervical samples were comparable to those for p16^{INK4a}. For small cell lung cancer only two of the 10 cases under investigation showed positivity for 14^{AFR} in hybrid capture. This result could be confirmed by immuno-cytochemistry. In the LBC samples from the oral cavity p14ARF could be detected in 7 out of 10 cases in concordance with the immuno-cytochemical findings.
The results show that detection of cyclin-dependent kinase inhibitors in a hybrid capture testing format from LBC samples may be used for assessment of diagnosis of several cancer entities. The results suggest that the biochemical testing could be used as an adjunct or conjunct testing to a cytological testing.

### Example 5: Immunochemical analysis of protein levels of p16^{INK4a}, Her-2/Neu and p14^{ARF} in Liquid based Cytology samples from Urine, sputum, breast fine-needle aspirates and from cervical swabs.

10 cervical swabs with a cytological classification as HSIL, 10 sputum samples with cytologically diagnosed small cell lung cancer, 10 urine samples from individuals with diagnosed bladder tumors and 10 fine needle aspirates from individuals with diagnosed DCIS, all provided in PreservCyt™ medium were subjected to centrifugation of the cells and subsequent solubilization of the cells in a lysis medium. Afterwards ELISA based detection of expression level of cyclin-dependent kinase inhibitor p16^{INK4a}, of p14^{ARF} and of HER-2/Neu in solutions prepared from the cells contained in the swabs.
The ELISA testing was performed as follows:

### (A)Cell Lysis

Each 10 mL of the LBC samples were centrifuged to allow the cells to sediment. The cell pellet is [TIME ]dissolved in 700µl of of mtm lysis buffer lysis medium (2% Triton X-100, 0.4% SDS, 0.6mM PMSF in PBS) by mixing and incubating for 10 Min at 80°C. The lysates of the LBC samples were then centrifuged at 4°C (15 min at 28.000 x g (16.600rpm HighspeedCentrifuge JEC Multi RF)); Supernatant was transferred to a fresh tube. The supernatant may be stored at -20 °C.

### (B) Performing the ELISA

### Coating of ELISA-plates

For each protein separate ELISA plates were prepared as follows. Stock-solutions of the primary antibodies specific for p16^{INK4a}, p14^{ARF} and HER-2/Neu were diluted in PBS to give ready-to-use coating solution.
For p16^{INK4a} clone mtm E6H4 was used for coating of ELISA plates. For p14^{ARF} polyclonal antibody directed against p14^{ARF} available from Calbiochem was used. For Her-2/Neu polyclonal antibody from DakoCytomation was used for coating.
50µl of the coating solution was added to each well of the ELISA plates.
For coating, the plates were incubated overnight at 4°C.
Coating solution was removed from the ELISA plates and the plates were rinsed using an automated ELISA washer as follows:
· 7 x 250µl washing buffer (0.1% Tween20 (v/v) in PBS) after removing remnants of the washing buffer, 300µl blocking buffer (2% BSA in PBS) was added to each well. Plates were incubated for 1h on a rocking device at ambient temperature.

### Incubation with samples

After removing the blocking buffer, 100µl of the lysed cell sample was added to each well.
For purpose of calibration of the test, different concentrations of recombinant proteins (0 pg/ml, 50 pg/ml, 100 pg/ml, 200 pg/ml, 400 pg/ml, 800 pg/ml) were included in each of the tests.
Samples were incubated for 1 h at room temperature.
Thereafter washing was performed on an automated ELISA washer as follows:
· 7 x 250µl washing buffer. The remaining buffer was removed.

### Incubation with detection antibody

Working solution of biotinylated secondary antibodies specific for the respective proteins were prepared by dilution of stock solution. For p16^{INK4a} mtm clone D7D7 was applied, for p14^{ARF} monoclonal antibody from Calbiochem was applied and for HER-2/Neu monoclonal Antibody from DakoCytomation was used.
100µl of working solution was added to each well. After incubation for 1h at RT, antibody solution was removed and ELISA plates were washed by an automated ELISA washer
· 7 x with 250µl washing buffer.

### Detection

Streptavidin-HRP-polymers (1mg/ml) were pre-diluted 1:10. (4µl +36µl incubation buffer); Final incubation solution was prepared by dilution 1:300 in incubation buffer (0,1% BSA in PBS) to a final concentration of 0,33 µg/ml.
100µl of this solution were added to each well and incubated for 1 h at RT.
Thereafter, the buffer was removed and the plates were washed manually with 200 µl washing buffer per well 5 times.

### Substrate incubation

TMB-substrate was equilibrated to 25°C for 1h in the dark.
100µl of substrate solution was added to each well.
The ELISA plates were incubated at 25°C for exactly 15 min in the dark. Then the reaction was stopped by addition of 80 µl 2,5M H₂SO₄.
Within 5 min. after stopping the reaction, OD 450 nm was determined. After evaluation of the results, each sample returned a value for the OD. For each antibody a threshold OD was determined using the value seen for background.
The ELISA results were compared to the diagnostic results of the cytological evaluation of the specimens from the same individuals. The results are as follows:

**Table 7:**

| **Cervical Samples** | | |
|---|---|---|
| **Cytological Diagnosis:** | | **10 HSIL** |
| **Immuno-Cytological Evaluation** | | |
| **p16^{INK4a}** | pos. | 10 |
| | neg. | 0 |
| **p14^{ARF}** | pos. | 8 |
| | neg. | 2 |
| **Her-2/Neu** | pos. | 2 |
| | neg. | 8 |

| **ELISA Evaluation** neg. 8 | | |
|---|---|---|
| **p16^{INK4a}** | pos. | 10 |
| | neg. | 0 |
| **p14^{ARF}** | pos. | 9 |
| | neg. | 1 |
| **Her-2/Neu** | pos. | 3 |
| | neg. | 7 |

| **Bladder Samples** | | |
|---|---|---|
| **Cytological Diagnosis:** | | **10 Carcinomas** |
| **Immuno-Cytological Evaluation** | | |
| **p16^{INK4a}** | pos. | 0 |
| | neg. | 10 |
| **p14^{ARF}** | pos. | 1 |
| | neg. | 9 |
| **Her-2/Neu** | pos. | 6 |
| | neg. | 4 |

| **ELISA Evaluation** | | |
|---|---|---|
| **p16^{INK4a}** | pos. | 0 |
| | neg. | 10 |
| **p14^{ARF}** | pos. | 0 |
| | neg. | 10 |
| **Her-2/Neu** | pos. | 5 |
| | neg. | 5 |

| **DCIS Samples** | | |
|---|---|---|
| **Cytological Diagnosis:** | | **10 DCIS** |
| **Immuno-Cytological Evaluation** | | |
| **p16^{INK4a}** | pos. | 0 |
| | neg. | 10 |
| **p14^{ARF}** | pos. | 0 |
| | neg. | 10 |
| **Her-2/Neu** | pos. | 6 |
| | neg. | 4 |

| **ELISA Evaluation** | | |
|---|---|---|
| **p16^{INK4a}** | pos. | 0 |
| | neg. | 10 |
| **p14^{ARF}** | pos. | 1 |
| | neg. | 9 |
| **Her-2/Neu** | pos. | 7 |
| | neg. | 3 |

It turned out that for p16^{INK4a} in 100% of the tested cases there was good correlation between the cytologically assessed p16^{INK4a} staining pattern and p16^{INK4a} positivity in an ELISA testing format using solubilized preservCyt™ samples for the analysis. For p14^{ARF} the correlation was 93%. For Her-2/Neu a correlation of more than 90% between the immuno-cytochemical detection of the overexpression and the positivity in the ELISA format could be detected.
The results of the above examples show that the biochemical testing format using solubilized LBC samples may be applied on the same specimens as the immuno-cytochemical analysis. As the biochemical testing consumes only a fraction of the LBC sample it may easily applied as an adjunct to the immuno-cytochemical anlysis.
There is good correlation between the immuno-cytochemical results and the ELISA results. This shows that the method according to the present invention is suited to assess diagnosis in various kinds of medically relevant conditions where liquid based cytology is currently applied either as ajunct or conjunct testing or as the case may be as a stand alone diagnostic test.

### Example 6: Immunochemical and RT-PCR analysis of mRNA/protein levels of MCM-5 and MCM-2 in Liquid based Cytology samples from Urine.

20 LBC samples of urine cells in CytoLyt™ were used for the present example. RT-PCR was performed in the same way as given in Example 3. Protein analysis was performed in a strip test foramta as given in Example 2 and in parallel in an ELISA format as given in Example 1. Experimental procedures were performed as given in these examples.
It could be shown that MCM-5 may easyliy be detected in lysates from urine LBC samples. The results obtained by the biochemical non-cell based assay on the protein as well as on the nucleic acid level corresponds pretty good to the results obtained from cytology. In cytology immuno-cytological staining for MCM-5 protein was used as aid in assessment of diagnosis.

## Claims

1. A method for assessment of diagnosis of a tumor selected from the group consisting of tumors of the anogenital tract, tumors of the gastrointestinal tract, tumors of the respiratory tract and tumors of the urinary system from solubilized Liquid based cytology (CBC) samples consisting of the steps of
a. detection of the presence or absence and/or the level of a marker polypeptide selected from the group of p16^{INK4a}, claudin, Ki67, KiS1, PCNA MCM-2, MCM-5, CDC-6, Her-2/Neu, Cyclin E and a HPV polypeptide derived from a HPV gene selected from the group consisting of HPV L1, HPV L2, HPV E1, HPV E2, HPV E4, HPV E5, HPV E6 and HPV E7;
b. comparing the presence or absence and/or the level of said marker polypeptide to the presence or absence and/or the level known to be characteristic for a healthy non-diseased body sample; and
c. assessing diagnosis of the tumor based on the presence or absence and/or the level of said marker polypeptide in the cells contained in the LBC sample compared to a healthy non-diseased body sample.

2. The method of Claim 1, wherein the tumor of the anogenital tract is cervical cancer, cervical intraepithelial neoplasia or cervical carcinoma in-situ.

3. The method of Claim 1, wherein the tumor of the gastrointestinal tract is colon cancer, cancer of the duodenum, pancreatic cancer, esophageal cancer, liver cancer, cancer of the biliary system or cancer of the anus or rectum.

4. The method of Claim 1, wherein the tumor of the respiratory tract is cancer of the lung, cancer of the alveoles, cancer of the bronchic tree, cancer of the nasopharyngeal space, cancer of the oral cavity, cancer of the lung, non-small cell lung cancer, adeno-squamous lung carcinoma, mesothelioma, carcinoid tumor of the lung or bronchial gland tumor.

5. The method of Claim 1, wherein the tumor of the urinary system is bladder cancer, cancer of the kidney, cancer of the renal pelvis, cancer of the ureters or cancer of the urethra.

6. The method according to any one of Claims 1 - 5, wherein the detection of a marker polypeptide is performed using at least one antibody, antibody fragment or miniantibody, specific for a the said marker polypeptide to be detected.

7. The method according to claim 6, wherein the antibody, antibody fragment or miniantibody is detectably labeled.

8. The method according to claim 7, wherein the label is selected from the group consisting of a radioisotope, a bioluminescent compound, a chemiluminescent compound, an electroluminescent compound, a fluorescent compound, a metal chelate, an enzyme, or a biologically relevant binding structure.

## Patentansprüche

1. Verfahren zur Diagnose eines Tumors, ausgewählt aus einer Gruppe umfassend Tumoren des Anogenitaltrakts, des Gastrointestinaltrakts, des Respirationstrakts und der Harnwege auf Basis von aufgelösten Dünnschicht-Zytologie-Proben (LBC) bestehend aus folgenden Schritten:
a. Nachweis der Anwesenheit oder Abwesenheit und/oder des Level eines Marker-Polypeptids ausgewählt aus einer Gruppe umfassend p16^{INK4a}, Claudin, Ki67, KiS1, PCNA, MCM-2, MCM-5, CDC-6, Her-2/Neu, Cyclin E und eines HPV-Polypeptids aus einem HPV-Gen ausgewählt aus einer Gruppe umfassend HPV L1, HPV L2, HPV E1, HPV E2, HPV E4, HPV E5, HPV E6 und HPV E7;
b. Vergleich der Anwesenheit oder Abwesenheit und/oder des Level des besagten Polypeptids mit der Anwesenheit oder Abwesenheit und/oder des Level charkteristisch für eine gesunde Körperprobe; und
c. Diagnose des Tumors basierend auf der Anwesenheit oder Abwesenheit und/oder des Level des besagten Marker-Polypeptids in den Zellen der Dünnschicht-Zytologie-Probe (LBC) im Vergleich zu einer gesunden Körperprobe.

2. Verfahren gemäß Anspruch 1, wobei der Tumor im Anogenitaltrakt ein Zervixkarzinom, eine zervikale intraepitheliale Neoplasie oder ein Zervixkarzinom In-situ ist.

3. Verfahren gemäß Anspruch 1, wobei der Tumor im Gastrointestinaltrakt ein Karzinom des Darmes, des Duodenums, der Bauchspeicheldrüse, der Speiseröhre, der Leber, der Gallenwege, des Afters oder des Rektums ist.

4. Verfahren gemäß Anspruch 1, wobei der Tumor im Respirationstrakt ein Karzinom der Lunge, der Alveolen, des Bronchialbaums, des Nasen-Rachenbereichs, der Mundhöhle, der Lunge, ein nicht-kleinzelliges Lungenkarzinom, ein adeno-squamöses Lungenkarzinom, ein Mesotheliom, ein Karzinoid-Tumor der Lunge oder ein Tumor der Bronchialdrüsen ist.

5. Verfahren gemäß Anspruch 1, wobei der Tumor der Harnwege ein Karzinom der Blase, der Nieren, des Nierenbeckens, der Harnleiter oder der Harnröhre ist.

6. Verfahren gemäß einem beliebigen der Ansprüche 1 - 5, wobei der Nachweis eines Marker-Polypeptids mit mindestens einem Antikörper, Antikörperfragment oder Miniantikörper erfolgt, welche spezifisch für besagtes nachzuweisendes Marker-Polypeptid sind.

7. Verfahren gemäß Anspruch 6, wobei der Antikörper, das Antikörperfragment oder der Miniantikörper nachweisbar markiert sind.

8. Verfahren gemäß Anspruch 7, wobei die Markierung ausgewählt ist aus einer Gruppe bestehend aus einem Radioisotop, einer biolumineszenten Verbindung, einer chemilumineszenten Verbindung, einer elektrolumineszenten Verbindung, einer fluoreszenten Verbindung, einem Metall-Chelat, einem Enzym oder einer biologisch elevanten Bindungsstruktur.

## Revendications

1. Procédé pour l'évaluation du diagnostic d'une tumeur sélectionnée parmi les tumeurs de l'appareil anogénital, les tumeurs de l'appareil gastro-intestinal, les tumeurs de l'appareil respiratoire et les tumeurs de l'appareil urinaire à partir d'échantillons solubilisés de cytologie en phase liquide, comprenant les étapes de :
a. détection de la présence ou de l'absence et/ou du taux d'un polypeptide marqueur appartenant au groupe comprenant p16^{INK4a}, claudine, Ki67, KiS1, PCNA MCM-2, MCM-5, CDC-6, Her-2/Neu, cycline E et un polypeptide du HPV dérivé d'un gène du HPV sélectionné parmi HPV L1, HPV L2, HPV E1, HPV E2, HPV E4, HPV E5, HPV E6 et HPV E7 ;
b. comparaison de la présence ou de l'absence et/ou du taux dudit polypeptide marqueur à la présence ou l'absence et/ou au taux connu comme étant caractéristique d'un échantillon corporel sain non pathologique ; et
c. évaluation du diagnostic de la tumeur en fonction de la présence ou de l'absence et/ou du taux dudit polypeptide marqueur dans les cellules contenues dans l'échantillon de cytologie en phase liquide par rapport à un échantillon corporel sain non pathologique.

2. Procédé selon la revendication 1, dans lequel la tumeur de l'appareil anogénital est un cancer du col de l'utérus, une néoplasie intraépithéliale cervicale ou un carcinome *in situ* du col de l'utérus.

3. Procédé selon la revendication 1, dans lequel la tumeur de l'appareil gastro-intestinal est un cancer du côlon, un cancer du duodénum, un cancer du pancréas, un cancer de l'oesophage, un cancer du foie, un cancer des voies biliaires ou un cancer de l'anus ou du rectum.

4. Procédé selon la revendication 1, dans lequel la tumeur de l'appareil respiratoire est un cancer du poumon, un cancer des alvéoles, un cancer de l'arbre bronchique, un cancer de l'espace nasopharyngé, un cancer de la cavité buccale, un cancer du poumon, un cancer du poumon non à petites cellules, un carcinome adéonosquameux du poumon, un mésothéliome, une tumeur carcinoïde du poumon ou un cancer glandulaire bronchique.

5. Procédé selon la revendication 1, dans lequel la tumeur de l'appareil urinaire est un cancer de la vessie, un cancer du rein, un cancer du bassinet rénal, un cancer des uretères ou un cancer de l'urètre.

6. Procédé selon l'une quelconque des revendications 1 à 5, dans lequel la détection d'un polypeptide marqueur est réalisée à l'aide d'au moins un anticorps, fragment d'anticorps ou minianticorps spécifique dudit polypeptide marqueur à détecter.

7. Procédé selon la revendication 6, dans lequel l'anticorps, le fragment d'anticorps ou le minianticorps est marqué de façon détectable.

8. Procédé selon la revendication 7, dans lequel le label est sélectionné parmi un radio-isotope, un composé bioluminescent, un composé chimioluminescent, un composé électroluminescent, un composé fluorescent, un chélate de métal, une enzyme ou une structure liante ayant une pertinence biologique.
